(19) Europäisches Patentamt
European Patent Office
Office européen des brevets

(11) Veröffentlichungsnummer: **0 483 582 A1**

# (12) EUROPÄISCHE PATENTANMELDUNG

(21) Anmeldenummer: **91117518.0**

(22) Anmeldetag: **14.10.91**

(51) Int. Cl.⁵: **C07D 495/04**, C07D 333/32, A61K 31/38, A01N 43/90, //(C07D495/04,333:00,311:00)

(30) Priorität: **25.10.90 DE 4033904**
**10.04.91 DE 4111652**

(43) Veröffentlichungstag der Anmeldung:
**06.05.92 Patentblatt 92/19**

(84) Benannte Vertragsstaaten:
**AT BE CH DE DK ES FR GB GR IT LI LU NL SE**

(71) Anmelder: **BAYER AG**

**W-5090 Leverkusen 1 Bayerwerk(DE)**

(72) Erfinder: **Bertram, Heinz-Jürgen, Dr.**
**Schneckenbergstrasse 18**
**W-5450 Holzminden(DE)**
Erfinder: **Lürssen, Klaus, Dr.**
**August-Kierspel-Strasse 145**
**W-5060 Bergisch Gladbach 2(DE)**
Erfinder: **Santel, Hans-Joachim, Dr.**
**Grünstrasse 9a**
**W-5090 Leverkusen 1(DE)**
Erfinder: **Schmidt, Robert R., Dr.**
**Im Waldwinkel 110**
**W-5060 Bergisch Gladbach 2(DE)**
Erfinder: **Plant, Andrew, Dr.**
**Im Wiesengrund 1b**
**W-5068 Odenthal(DE)**
Erfinder: **Krüger, Bernd-Wieland, Dr.**
**Unterboschbach 19**
**W-5060 Bergisch Gladbach 2(DE)**
Erfinder: **Harder, Achim, Dr.**
**Piccoloministrasse 398**
**W-5000 Köln 80(DE)**
Erfinder: **Mencke, Norbert, Dr.**
**Grundermühle 2**
**W-5090 Leverkusen 3(DE)**

## (54) Thieno[3,2-b]pyranone, Verfahren zu ihrer Herstellung, Zwischenprodukte, ihre Verwendung zur Bekämpfung von Parasiten und zur Regulierung des Pflanzenwachstums.

(57) Neuartige substituierte Thieno[3,2-b]pyranone allgemeinen Formel (I)

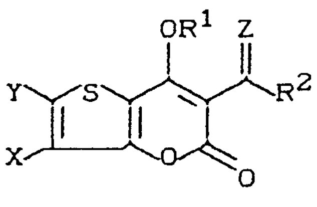

(I)

EP 0 483 582 A1

Verfahren zu ihrer Herstellung und ihre Verwendung als Herbizide und Pflanzen-Wachstumsregulatoren sowie neuartige Zwischenprodukte der Formel (VI)

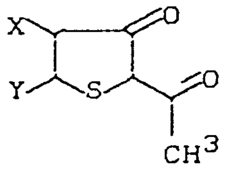

(VI)

(worin $R^1$, $R^2$, X, Y und Z jeweils die in der Beschreibung angegebenen Bedeutungen haben).

Die vorliegende Erfindung betrifft neuartige, substituierte Thieno[3,2-b]pyran-5,7-dione Verfahren und neuartige Zwischenprodukte zu ihrer Herstellung sowie ihre Verwendung als Endoparasitizide, Herbizide und als Pflanzenwachstumsregulatoren.

Es ist bekannt, daß bestimmte Thieno[3,2-b]pyran-5,7-dione herbizide Eigenschaften besitzen (vgl. EP-A-0 345 635).

Es wurden nun neuartige substituierte Thieno[3,2-b]pyran-5,7-dione dione der allgemeinen Formel (I) und ihrer tautomeren Formen

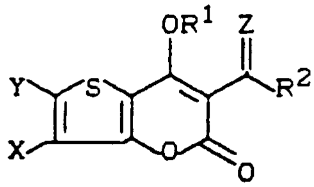

(I)

gefunden, in welcher

$R^1$ für Wasserstoff, geradkettige oder verzweigte Reste aus der Reihe Alkyl, Alkenyl, Alkinyl Alkylsulfonyl, Alkylcarbonyl, Alkenylcarbonyl, jeweils substituiertes oder unsubstituiertes Phenyl, Benzoyl oder Benzoylsulfonyl steht,

$R^2$ für geradkettige oder verzweigte Reste aus der Reihe Alkyl, Alkenyl, Alkinyl, Halogenalkyl, Cycloalkyl, jeweils substituiertes oder unsubstituiertes Phenyl oder Benzyl steht,

X und Y unabhängig voneinander für Wasserstoff, Halogen, Cyano, Nitro, geradkettiges oder verzweigtes Alkyl, Alkenyl, Alkinyl, Halogenalkyl, Cycloalkyl, Alkylcarbonyl, Alkoxycarbonyl, Alkyloxy, Alkylthio, Halogenalkoxy, Halogenalkylthio, Alkylamino, Dialkylamino, jeweils substituiertes oder unsubstituiertes Aryl, Aralkyl, Aryloxy oder Arylthio stehen, oder

X und Y gemeinsam mit den angrenzenden C-Atomen einen carbocyclischen Ring bilden, der gegebenenfalls durch Heteroatome unterbrochen ist und

Z für Sauerstoff oder $=NOR^3$ steht,

wobei

$R^3$ für Wasserstoff und geradkettige oder verzweigte Reste aus der Reihe Alkyl, Alkenyl, Alkinyl, Halogenalkyl, Halogenalkenyl, Halogenalkinyl, Cycloalkyl, substituiertes oder unsubstituiertes Phenyl, Phenylalkyl steht,

sowie deren verträgliche Salze in Frage kommen.

Weiterhin wurde gefunden, daß man die neuartigen Thieno-[3,2-b] pyran-5,7 -dione der allgemeinen Formel (I) und ihre tautomeren Formen

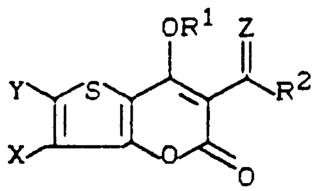

(I)

in welcher

$R^1$ für Wasserstoff, geradkettige oder verzweigte Reste aus der Reihe Alkyl, Alkenyl, Alkinyl Alkylsulfonyl, Alkylcarbonyl, Alkenylcarbonyl, jeweils substituiertes oder unsubstituiertes Phenyl, Benzoyl oder Benzoylsulfonyl steht,

$R^2$ für geradkettige oder verzweigte Reste aus der Reihe Alkyl, Alkenyl, Alkinyl, Halogenalkyl, Cycloalkyl, jeweils substituiertes oder unsubstituiertes Phenyl oder Benzyl steht,

X und Y unabhängig voneinander für Wasserstoff, Halogen, Cyano, Nitro, geradkettiges oder verzweigtes Alkyl, Alkenyl, Alkinyl, Halogenalkyl, Cycloalkyl, Alkylcarbonyl, Alkoxycarbonyl, Alkyloxy, Alkylthio, Halogenalkoxy, Halogenalkylthio, Alkylamino, Dialkylamino, jeweils substituiertes oder unsubstituiertes Aryl, Aralkyl, Aryloxy oder Arylthio stehen, oder

X und Y gemeinsam mit den angrenzenden C-Atomen einen carbocyclischen Ring bilden, der gegebenenfalls durch Heteroatome unterbrochen ist und

Z für Sauerstoff oder $NOR^3$ steht, wobei

$R^3$ für Wasserstoff und geradkettige oder verzweigte Reste aus der Reihe Alkyl, Alkenyl, Alkinyl, Halogenalkyl, Halogenalkenyl, Halogenalkinyl, Cycloalkyl, substituiertes oder unsubstituiertes Phenyl oder Phenylalkyl steht,

sowie deren verträgliche Salze erhält, wenn man

a) Thieno[3,2-b]pyran-5,7-dione der Formel (II)

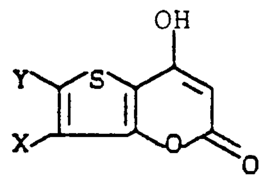

(II)

in welcher

X und Y die oben angegebene Bedeutung haben,

mit einem Säurechlorid der Formel (III)

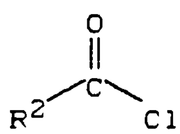

(III)

in welcher

$R^2$ die oben angegebene Bedeutung hat,

gegebenenfalls in Gegenwart einer Base und gegebenenfalls in Gegenwart eines Verdünnungsmittels zum Enolester umsetzt und anschließend gegebenenfalls in Gegenwart eines Reaktionshilfsmittels im gleichen oder in einem anderen Verdünnungsmittel bei einer Temperatur zwischen 0 °C und 100 °C zur C-Acylierung bringt, oder

b) wenn man die Thieno[3,2-b]pyran-2,4-dione gemäß der allgemeinen Formel (II) mit einer Carbonsäure der allgemeinen Formel (IV)

$R^2$-COOH (IV)

in welcher

$R^2$ die oben angegebene Bedeutung hat,

gegebenenfalls in Gegenwart eines Verdünnungsmittels und gegebenenfalls in Gegenwart eines wasserabspaltenden Reagenz und gegebenenfalls in Gegenwart eines Reaktionshilfsmittels bei einer Temperatur zwischen 0 °C und 100 °C umsetzt,

oder wenn man

c) Thienopyran-2,4-dione der Formel (II) mit Säurechloriden der Formel (III) in Gegenwart einer Lewis-Säure in einem Reaktionsschritt zu Derivaten der allgemeinen Formel (I), in welcher Z für Sauerstoff steht und $R^1$ für Wasserstoff steht,

umsetzt,

d) oder wenn man für den Fall, daß in Formel (I) Z für $=NOR^3$ steht Thieno[3,2-b]pyran-5,7-dione der Formel (I)

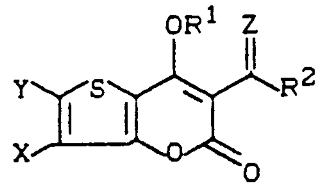

(I)

in der

Z für Sauerstoff steht und die übrigen Reste die oben angegebene Bedeutung haben,

mit einem entsprechenden Hydroxylamin oder seinen Ammoniumverbindungen $R^3ONH_2$ oder $R^3ONH_3^{\oplus}W^{\ominus}$, worin $W^{\ominus}$ für ein Anion-Äquivalent einer anorganischen Säure steht und $R^3$ die oben angegebene Bedeutung hat, bei einer Temperatur zwischen 0 °C und 80 °C umsetzt, wobei man die Thieno[3,2-b]pyran-5,7-dion-Derivate der Formel (I) erhält, in denen Z eine Oximethergruppe (= $NOR^3$) bedeutet.

e) oder wenn man für den Fall,

daß Verbindungen der Formel (I), in der $R^1$ für geradkettige oder verzweigte Reste aus der Reihe Alkyl, Alkenyl, Alkinyl, Alkylsulfonyl, Alkylcarbonyl, Alkenylcarbonyl, jeweils substituiertes oder unsubstituiertes Phenyl, Benzoyl oder Benzoylsulfonyl steht, und $R^2$, X Y und Z die oben genannte Bedeutung haben, erhalten werden sollen Verbindungen der Formel (I), in welcher

$R^1$ für Wasserstoff steht

mit Verbindungen der Formel (IX)

$R^1$- V (IX)

in welcher

$R^1$ für geradkettige oder verzweigte Reste aus der Reihe Alkyl, Alkenyl, Alkinyl, Alkylsulfonyl, Alkylcarbonyl, Alkenylcarbonyl, jeweils substituiertes oder unsubstituiertes Phenyl, Benzoyl oder Benzoylsulfonyl steht und

V für Halogen, insbesondere Chlor, Brom oder Jod steht,

umgesetzt werden.

Die Verbindungen der Formel (I) lassen sich hervorragend als Endoparasitizide insbesondere auf dem Gebiet der Veterinärmedizin einsetzen.

Schließlich wurde gefunden, daß die neuartigen Thieno[3,2-b] pyran-5,7-dione der allgemeinen Formel (I), in denen Z eine Oximethergruppe (= $NOR^3$) bedeutet, eine unerwartete, herbizide Wirkung besitzen und Derivate, in denen Z Sauerstoff bedeutet, pflanzenwachstumsregulierende Eigenschaften haben.

Die Erfindung betrifft bevorzugt Verbindungen der Formel (I) und ihre tautomeren Formen, in welcher

$R^1$ für Wasserstoff, geradkettige oder verzweigte Reste aus der Reihe $C_1$-$C_7$-Alkyl, $C_3$-$C_6$-Alkenyl, $C_3$-$C_6$-Alkinyl, $C_1$-$C_6$-Alkylsulfonyl, $C_1$-$C_6$-Alkylcarbonyl, $C_2$-$C_6$-Alkenylcarbonyl, jeweils substituiertes oder unsubstituiertes Phenyl, Benzolsulfonyl oder Benzoyl steht,

$R^2$ für geradkettige oder verzweigte Reste aus der Reihe $C_1$-$C_7$-Alkyl, $C_2$-$C_6$-Alkenyl, $C_2$-$C_6$-Alkinyl, $C_1$-$C_7$-Halogenalkyl mit 1 bis 15 gleichen oder verschiedenen Halogenatomen, $C_3$-$C_7$-Cycloalkyl oder jeweils substituiertes oder unsubstituiertes Phenyl oder Benzyl steht,

X und Y unabhängig voneinander für Wasserstoff, Halogen, Cyano, Nitro, geradkettiges oder verzweigtes $C_1$-$C_7$-Alkyl, $C_2$-$C_6$-Alkenyl, $C_2$-$C_6$-Alkinyl, $C_1$-$C_7$-Halogenalkyl mit 1 bis 15 gleichen oder verschiedenen Halogenatomen, $C_3$-$C_6$-Cycloalkyl, $C_1$-$C_6$-Alkylcarbonyl, $C_1$-$C_6$-Alkyloxycarbonyl, $C_1$-$C_7$-Alkyloxy, $C_1$-$C_7$-Alkylthio, 1-5 Halogen-$C_{1-4}$-alkoxy, 1-5 Halogen-$C_{1-4}$-alkylthio, $C_1$-$C_7$-Alkylamino, $C_1$-$C_7$-Dialkylamino, jeweils substituiertes oder unsubstituiertes Aryl, Aralkyl, Aryloxy oder Arylthio stehen, oder

X und Y gemeinsam einen carbocyclischen Ring bestehend aus 4 bis 8 Kohlenstoffatomen, der gegebenenfalls durch Heteroatome, wie Stickstoff, Sauerstoff oder Schwefel unterbrochen ist, bilden und

Z für Sauerstoff oder $NOR^3$ steht, wobei

$R^3$ für Wasserstoff und geradkettige oder verzweigte Reste aus der Reihe $C_1$-$C_7$-Alkyl, $C_2$-$C_6$-Alkenyl, $C_2$-$C_6$-Alkinyl, $C_1$-$C_7$-Halogenalkyl mit 1 bis 15 gleichen oder verschiedenen Halogenatomen, $C_2$-$C_6$-Halogenalkenyl mit 1 bis 11 gleichen oder verschiedenen Halogenatomen, $C_2$-$C_6$-Halogenalkinyl mit 1 bis 9 gleichen oder verschiedenen Halogenatomen, $C_3$-$C_6$-

Cycloalkyl, oder substituiertes oder unsubstituiertes Phenyl oder Benzyl steht,
wobei als Substituenten an den Aromaten Halogen, Cyano, Nitro, geradkettiges oder verzweigtes Alkyl, Halogenalkyl, Alkoxy, Halogenalkoxy, Alkylthio, Halogenalkylthio, Alkylsulfinyl, Halogenalkylsulfinyl, Alkylsulfonyl, Halogenalkylsulfonyl, Alkylendioxy, Halogenalkylendioxy in Frage kommen,
sowie deren verträgliche Salze.

Die Erfindung betrifft besonders bevorzugt Verbindungen der Formel (I) und ihre tautomeren Formen, in welcher

$R^1$ für Wasserstoff, geradkettige oder verzweigte und Reste aus der Reihe $C_1$-$C_6$-Alkyl, $C_3$-$C_4$-Alkenyl, $C_3$-$C_4$-Alkinyl, $C_1$-$C_4$-Alkylsulfonyl, $C_1$-$C_4$-Alkylcarbonyl, $C_2$-$C_5$-Alkenylcarbonyl, oder jeweils substituiertes oder unsubstituiertes Phenyl, Benzolsulfonyl oder Benzoyl steht,

$R^2$ für geradkettige oder verzweigte Reste aus der Reihe $C_1$-$C_6$-Alkyl, $C_1$-$C_4$-Alkenyl, $C_2$-$C_4$-Alkinyl, $C_1$-$C_6$-Halogenalkyl mit 1 bis 13 gleichen oder verschiedenen Halogenatomen oder $C_3$-$C_6$-Cycloalkyl, jeweils substituiertes oder unsubstituiertes Phenyl oder Benzyl steht,

X und Y unabhängig voneinander für Wasserstoff, Halogen, Cyano, Nitro, geradkettiges oder verzweigtes $C_1$-$C_6$-Alkyl, $C_2$-$C_5$-Alkenyl, $C_2$-$C_5$-Alkinyl, $C_1$-$C_6$-Halogenalkyl mit 1 bis 13 gleichen oder verschiedenen Halogenatomen, $C_3$-$C_6$-Cycloalkyl, $C_3$-$C_6$-Alkylcarbonyl, $C_1$-$C_6$-Alkyloxycarbonyl, $C_1$-$C_6$-Alkyloxy, $C_1$-$C_6$-Alkylthio, $C_1$-$C_6$-Alkylamino, $C_1$-$C_6$-Dialkylamino, jeweils substituiertes oder unsubstituiertes Aryl, Aralkyl, Aryloxy oder Arylthio stehen, oder

X und Y gemeinsam mit den angrenzenden C-Atomen einen carbocyclischen Ring bestehend aus 4 bis 8 Kohlenstoffatomen, der gegebenenfalls durch Heteroatome, wie Stickstoff, Sauerstoff oder Schwefel unterbrochen ist, bilden und

Z für Sauerstoff oder $NOR^3$ steht, wobei

$R^3$ für Wasserstoff und geradkettige oder verzweigte Reste aus der Reihe $C_1$-$C_6$-Alkyl, $C_2$-$C_4$-Alkenyl, $C_2$-$C_4$-Alkinyl, $C_1$-$C_6$-Halogenalkyl mit 1 bis 13 gleichen oder verschiedenen Halogenatomen, $C_2$-$C_4$-Halogenalkenyl mit 1 bis 7 gleichen oder verschiedenen Halogenatomen, $C_2$-$C_4$-Halogenalkinyl mit 1 bis 5 gleichen oder verschiedenen Halogenatomen, $C_3$-$C_6$-Cycloalkyl, oder substituiertes oder unsubstituiertes Phenyl oder Benzyl steht,

wobei als Substituenten an den Aromaten Halogen, Cyano, Nitro, geradkettiges oder verzweigtes $C_1$-$C_4$-Alkyl, 1-5-Halogen-$C_{1-4}$-alkyl $C_{1-4}$-Alkoxy, 1-5-Halogen-$C_{1-4}$-alkoxy, $C_{1-4}$-Alkylthio, 1-5-Halogen-$C_{1-4}$-alkylthio, $C_{1-4}$-Alkylsulfinyl, 1-5-Halogen-$C_{1-4}$-alkylsulfinyl, $C_{1-4}$-Alkylsulfonyl, 1-5-Halogen-$C_{1-4}$-alkylsulfonyl, $C_{1-2}$-Alkylendioxy, 1-4-Halogen-$C_{1-2}$-alkylendioxy in Frage kommen,
sowie deren verträgliche Salze mit einwertigen und zweiwertigen Metallkationen, jeweils substituierten oder unsubstituierten Ammoniumionen, Phosphoniumionen, Sulfoniumionen und Sulfoxoniumionen.

Die Erfindung betrifft insbesonders bevorzugt Verbindungen der Formel (I) und ihre tautomeren Formen, in welcher

$R^1$ für Wasserstoff, Methyl, Ethyl, n-Propyl, i-Propyl, n-Butyl, i-Butyl, t-Butyl, n-Pentyl, i-Pentyl, Allyl, n-Butenyl, i-Butenyl, Propinyl, Acetyl, Propionyl, Acroyl, Isobutyryl, Pivaloyl, Valeroyl, Phenyl, Benzoyl, 4-Methylbenzoyl, 4-Ethylbenzoyl, 4-n-Propylbenzoyl, 4-Isopropylbenzoyl, 4-n-Butylbenzoyl, 4-i-Butylbenzoyl, 4-t-Butylbenzoyl, 2,4,6-Trimethylbenzoyl, p-Methylphenylsulfonyl, Benzoylsulfonyl steht,

$R^2$ für Methyl, Ethyl, n-Propyl, i-Propyl, n-Butyl, s-Butyl, i-Butyl, t-Butyl, n-Pentyl, i-Pentyl, s-Pentyl, t-Pentyl, neo-Pentyl, n-Hexyl, Cyclopropyl, Cyclobutyl, Cyclopentyl, Cyclohexyl, jeweils substituiertes oder unsubstituiertes Phenyl oder Benzyl steht,

X und Y unabhängig voneinander für Wasserstoff, Fluor, Chlor, Brom, Cyano, Methyl, Ethyl, n-Propyl, Isopropyl, n-Butyl, i-Butyl, s-Butyl, t-Butyl, n-Pentyl, Cyclopropyl, Cyclobutyl, Cyclopentyl, Cyclohexyl, Methoxy, Ethoxy, n-Propyloxy, i-Propyloxy, n-Butyloxy-, i-Butyloxy, t-Butyloxy, n-Pentyloxy oder neo-Pentyloxy, Methylthio-, Ethylthio-, n-Propylthio, i-Propylthio, n-Butylthio-, i-Butylthio, t-Butylthio, n-Pentylthio, Dimethylamino, Diethylamino, Dipropylamino, Dibutylamino, Methoxymethyl, Ethoxymethyl, Methylthiomethyl, Ethylthiomethyl, 1-Methoxyethyl, 2-Methoxythio-ethyl, 1,3-Dimethoxypropyl, 2-Ethoxyethyl, 1-Methyl-2-methylthio-ethyl, 2-Methyl-1-methylthiomethylpropyl, 1-Methyl-butyl, 2-Methyl-1-propenyl, Phenyl, 4-Methylphenyl, 4-Trifluormethylphenyl, 2,4-Dichlorphenyl, 4-Methoxyphenyl, 2,4,6-Trimethylphenyl, 3,5-Dibromphenyl, 4-Methylthiophenyl, 4-Fluorphenyl stehen, oder

X und Y gemeinsam mit den angrenzenden C-Atomen einen carbocyclischen Ring bestehend aus 5 bis 6 Kohlenstoffatomen, der gegebenenfalls durch Heteroatome wie Stickstoff, Sauerstoff oder Schwefel unterbrochen ist, bilden und

Z für Sauerstoff oder $NOR^3$ steht, wobei

$R^3$ für Wasserstoff und Methyl, Ethyl, n-Propyl, i-Propyl, n-Butyl, i-Butyl, t-Butyl, Allyl, Prop-2-inyl, 3-Fluorpropyl, trans-3-Chlor-prop-2-enyl, trans-But-2-enyl, cis-3-Chlor-prop-2-enyl, But-2-inyl, 2-Methoxyethyl, E-4(4-Fluorphenyl)but-2-enyl, E-4(4-Chlorphenyl)but-2-enyl, E-4(4-tert.-Butylphenyl)but-2-enyl, E-4(4-Trifluorphenyl)but-2-enyl; E-4-Phenyl-but-2-enyl, 2-Thienyl, 3-Thienyl; 5-Chlor-thienyl und gegebenenfalls substituiertes Benzyl steht,
wobei als Substituenten an den Aromaten Halogen, Cyano, Nitro, Methyl, Ethyl, n-Propyl, i-Propyl, n-Butyl, i-Butyl, t-Butyl, Chlormethyl, Fluormethyl, Dichlormethyl, Trichlormethyl, Difluormethyl, Trifluormethyl, Trifluormethoxy, Trifluormethylthio, Methylsulfinyl, Methylsulfonyl, Trifluormethylsulfinyl, Trifluormethylsulfonyl in Frage kommen,

sowie deren verträgliche Salze z.B. mit einwertigen Metallkationen, insbesondere Natrium, Kalium, oder ein Äquivalent eines mehrwertigen Kations, z.B. ein Erdalkalimetallkation, insbesondere Magnesium, Calcium sowie landwirtschaftlich genutzte Kationen wie Mangan-, Kupfer-, Zink- und Eisenkationen sowie Ammonium-, Phosphonium-, Sulfonium- und Sulfoxoniumkationen wie Ammonium, Tetraalkylammonium, Benzyltrialkylammonium, Trialkylsulfonium oder Trialkylsulfoxonium.

Die oben aufgeführten allgemeinen oder in Vorzugsbereichen aufgeführten Restdefinitionen bzw. Erläuterungen gelten für Endprodukte und für die Ausgangs- und Zwischenprodukte entsprechend. Diese Restedefinitionen können untereinander, also auch zwischen den jeweiligen Vorzugsbereichen beliebig kombiniert werden.

Verwendet man beispielsweise Thieno-[3,2-b]pyran-5,7-dion und Buttersäurechloid als Edukte, so läßt sich der Reaktionsablauf des erfindungsgemäßen Verfahrens (a) durch das folgende Formelschema darstellen:

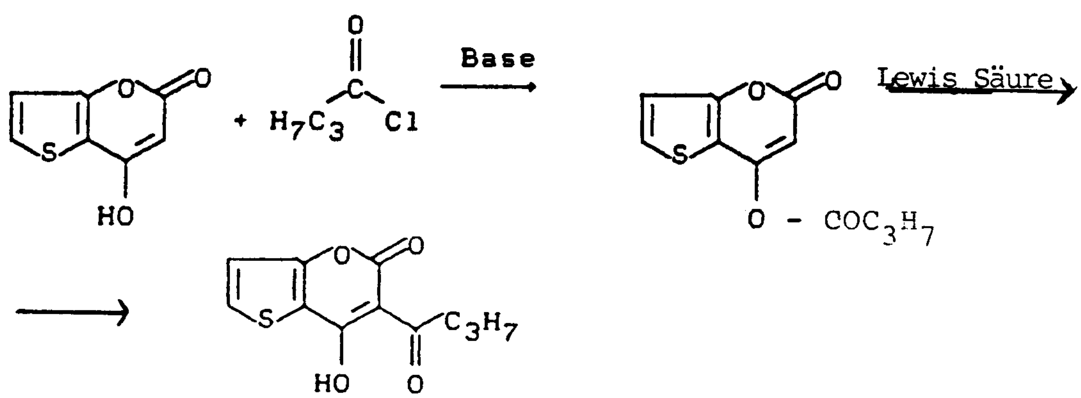


Dabei reagiert das Thieno-[3,2-b]pyran-5,7-dion zunächst zum Enolester und anschließend mit einem sauren oder basischen Katalysator im gleichen oder in einem anderen Lösungsmittel zum erfindungsgemäßen Thieno[3,2-b]-6-butyrylpyran-5,7-dion.

In Gegenwart eines sauren Katalysators erhält man in einem Reaktionsschritt das erfindungsgemäße Thieno-[3,2-b]-6-butyryl-pyran-5,7-dione (Verfahren c).

Verwendet man beipielsweise Thieno-[3,2-b]pyran-5,7-dion und Buttersäure als Edukte, so läßt sich der Reaktionsablauf des erfindungsgemäßen Verfahrens (b) durch das folgende Formelschema darstellen:

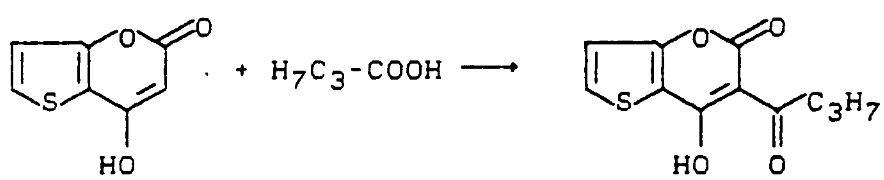

Das Thieno[3,2-b]pyran-5,7-dion reagiert in diesem Fall mit der jeweiligen Carbonsäure in Gegenwart eines wasserabspaltenden Reagenz wie z.B. anorganische Säurechloride und in Gegenwart eines Katalysators zum erfindungsgemäßen Thieno[3,2-b]pyran-5,7-dion.

Verwendet man beispielsweise Thieno[3,2-b]-6-butyryl-pyran-5,7-dion mit einer Carbonylgruppe in der Seitenkette und Hydroxylamin oder seine Ammoniumverbindung wie Ethoxyaminhydrochlorid als Edukte, so läßt sich der Reaktionsablauf des erfindungsgemäßen Verfahrens (d) durch das folgende Formelschema darstellen:

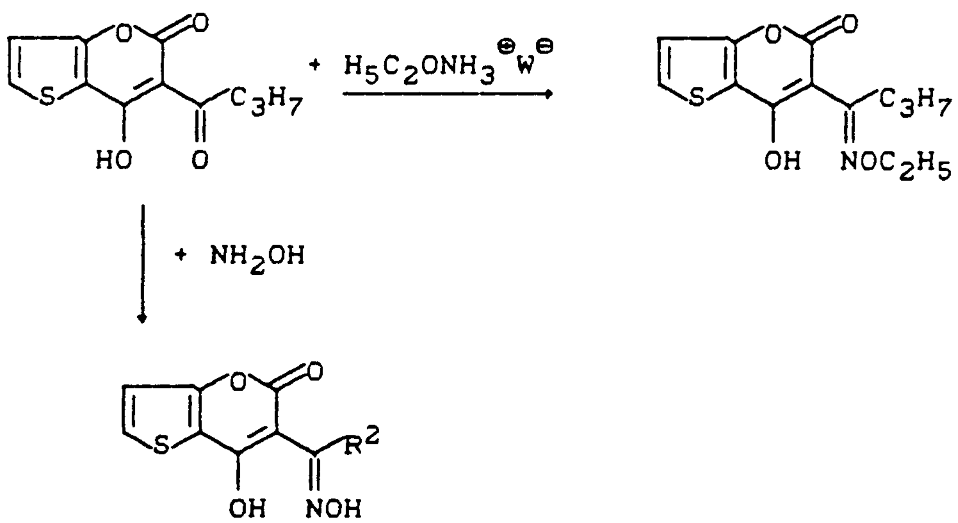


Verwendet man 8-Methyl-Thieno[3,2-b]-6-(1-ethoxyiminopropyl)pyran-5,7-dion und Methyliodid als Edukte, so läßt sich der Reaktionsablauf des erfindungsgemäßen Verfahrens (e) durch das folgende Formelschema darstellen:

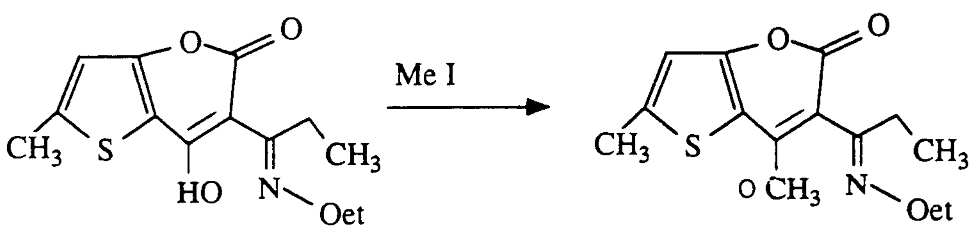


Die zur Durchführung der erfindungsgemäßen Verfahren (a), (b) und (c) als Edukte benötigten Thieno-[3,2-b]pyran-5,7-dione sind durch die Formel (II) allgemein definiert. In dieser Formel (II) stehen $R^1$, X und Y vorzugsweise für diejenigen Reste, die bereits im Zusammenhang mit der Beschreibung der erfindungsgemäßen Stoffe der Formel (I) als bevorzugt für diese Substituenten genannt werden. Die Thieno[3,2-b]pyran-5,7-dione der Formel (II) sind bekannt oder können nach an sich bekannten Verfahren aus 2-Acetyl-3-hydroxythiophenen (V) hergestellt werden. [T.Kraft, Recueil 86, 971 (1967)].

Die Darstellung der 2-Acetyl-3-hydroxythiophene (V) gelingt durch Umsatz von 2,5-Dihydroxy-2,5-dimethyl-1,4-dithian mit Propiolestern (vgl. T. Kraft, Recueil 86, 971 (1967)) oder durch Umsatz von 2,5-Dihydroxy-2,5-dimethyl-1,4-dithian mit α-Halogenacrylestern.

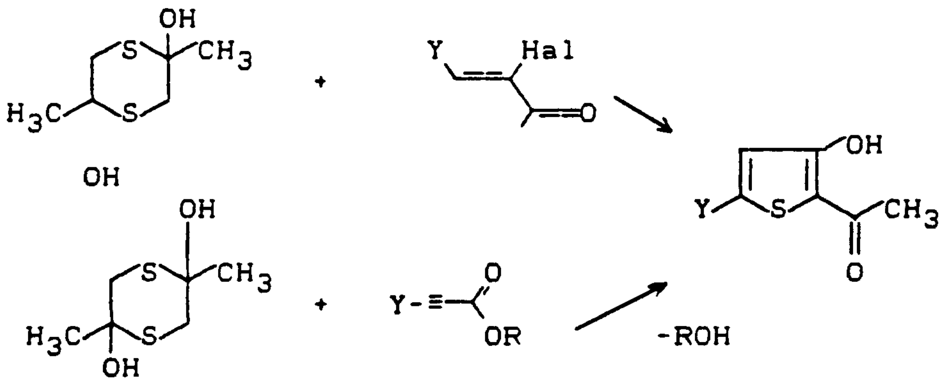


(V)

Hal = Halogen oder äquivalente Abgangsgruppe
Y = wie oben definiert
R = niedere Alkylreste

Alternnativ dazu ist die Darstellung von 2-Acetyl-3-hydroxy-thiophenen durch Umsetzung von $\beta$-Halogen-substituierten $\alpha,\beta$-ungesättigten Estern mit 2,5-Dihydroxy-2,5-dimethyl-1,4-dithion oder durch Umsetzung von $\beta$-Thioxoestern mit $\alpha$-Halogenaceton (DE 3 925 719, 1989) oder durch Dehydrierung von 2-Acetyl-3-keto-4,5-dihydrothiophen der Formel (VI) (DE 2 615 885, 1976) möglich.

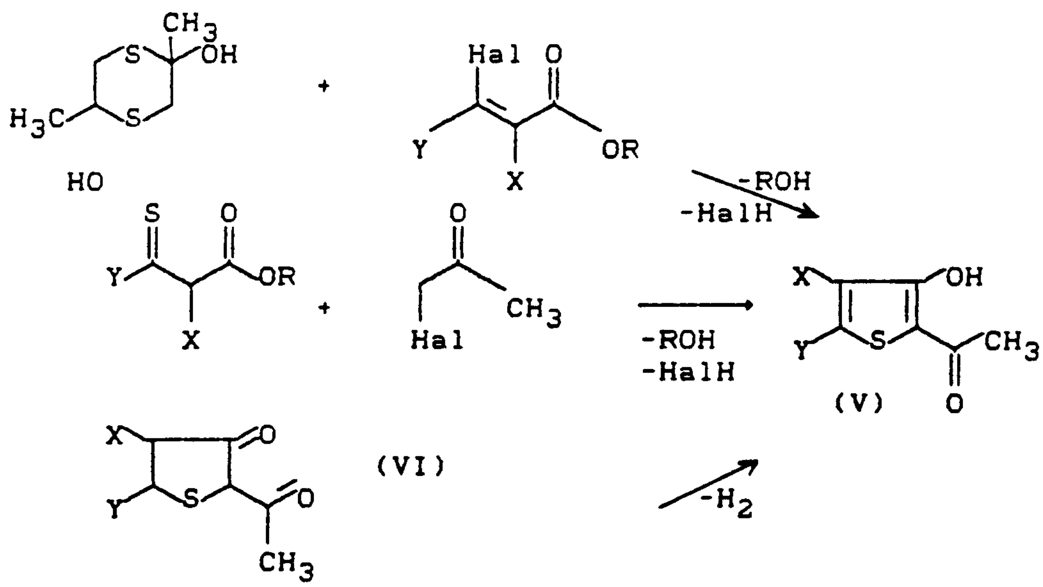


(V)

(VI)

Hal = Halogen oder äquivalente Abgangsgruppe
X, Y = wie oben definiert

Die $\beta$-Thioxoester sind bekannt [F. Duus, Tetrahedron 28, 5923 (1972)].

Die 2-Acetyl-3-keto-4,5-dihydrothiophene der Formel (VI)

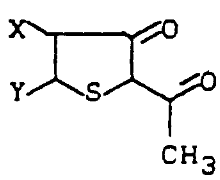

(VI)

X, Y = wie oben definiert
sind neu und ebenfalls Gegenstand der Erfindung. Sie können durch Umsetzung von 2,5-Dihydroxy-2,5-dimethyl1,4-dithian mit Acrylestern der Formel (VII) erhalten werden:

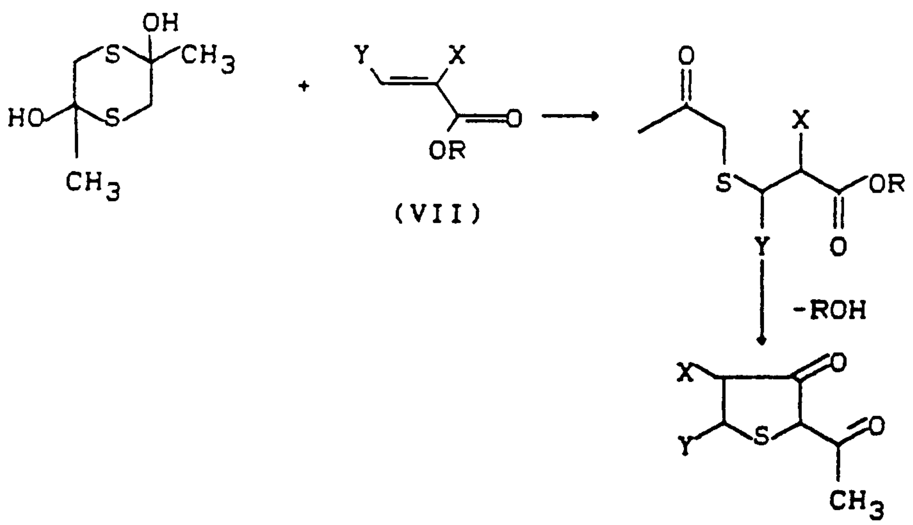


Die zur Durchführung des erfindungsgemäßen Verfahrens (a) und (c) weiterhin als Edukte benötigten Säurechloride sind durch die Formel (III) allgemein definiert, die zur Durchführung des erfindungsgemäßen Verfahrens (b) weiterhin als Edukte benötigten Carbonsäuren sind durch die Formel (IV) allgemein definiert. In diesen Formeln (III) und (IV) steht $R^2$ vorzugsweise für diejenigen Reste, die bereits im Zusammenhang mit der Beschreibung der erfindungsgemäßen Stoffe der Formel (I) als bevorzugt für diesen Substituenten genannten wurden. Die Säurehalogenide der Formel (III) und die Carbonsäuren der Formel (IV) sind allgemein bekannte Verbindungen der organischen Chemie.

Die zur Durchführung des Verfahrens (d) weiterhin als Edukte benötigten Halogenide sind durch die Formel (IX) allgemein definiert. In dieser Formel steht $R^1$ vorzugsweise für diejenigen Reste, die bereits im Zusammenhang mit der Beschreibung der erfindungsgemäßen Stoffe der Formel (I) als bevorzugt für $R^1$ genannt wurden, ausgenommen Wasserstoff. Die Verbindungen der Formel (IX) sind allgemein bekannte Verbindungen der organischen Chemie.

Die zur Durchführung des erfindungsgemäßen Verfahrens (c) als Edukte benötigten Thieno[3,2-b]pyran-5,7-dione sind durch die Formel (I) allgemein definiert. In dieser Formel (I) stehen $R^1$, $R^2$, X und Y vorzugsweise für diejenigen Reste, die bereits im Zusammenhang mit der Beschreibung der erfindungsgemäßen Stoffe der Formel (I) als bevorzugt für diese Substituenten genannt werden. Z steht jedoch im Falle des erfindungsgemäßen Verfahrens ( d) für eine Oximethergruppe ( $=NOR^3$), worin $R^3$ vorzugsweise für diejenigen Reste steht, die ebenfalls bereits im Zusammenhang mit der Beschreibung der erfindungsgemäßen Stoffe der Formel (I) als bevorzugt für diese Substituenten genannten werden:

Die Verbindungen der Formel (I), in denen $R^1$ Wasserstoff ist, können in mehreren, von der Formel (I) verschiedenen tautomeren Formen auftreten, die ebenfalls zur Erfindung gehören.

Für den Fall, daß olefinisch ungesättigte Substituenten vorhanden sind, können in bekannter Weise E- und Z-Isomere auftreten, die gleichermaßen zur Erfindung gehören.

Als Verdünnungsmittel zur Durchführung der erfindungsgemäßen Verfahren (a), (b), (c) und (e) komman inerte organische Lösungsmittel infrage. Hierzu gehören insbesondere aliphatische, alicyclische oder aromatische Kohlenwasserstoffe, wie beispielsweise Benzol, Toluol, Xylol, Petrolether, Hexan, Cyclohexan, Ether, wie Diethylether, Dioxan, Tetrahydrofuran oder Ethylenglykoldimethyl- oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid oder Sulfone, wie Sulfolan, Alkohole wie Methanol, Ethanol oder Isopropanol, chlorierte Kohlenwasserstoffe wie Chloroform oder Dichlormethan.

Die erfindungsgemäßen Verfahren (a), (b), (c) und (e) werden vorzugsweise in Gegenwart eines geeigneten Reaktionshilfsmittels durchgeführt. Als solche kommen alle üblicherweise verwendbaren anorganischen und organischen Basen in Frage. Vorzugsweise verwendet man Alkalimetallhydride, -hydroxide, -amide, -alkoholate, -carbonate oder -hydrogencarbonate, wie beispielsweise Natriumhydrid, Natriumamid,

Natriumhydroxid, Natriummethylat, Natriumethylat, Kalium-t-butylat, Natriumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU), Oxide von Erdalkalimetallen wie Magnesiumoxid oder Calciumoxid.

Die Base wird in stöchiometrischer Menge oder im Überschuß zugesetzt.

Als saure oder basische Katalysatoren für das erfindungsgemäße Verfahren (a), (c) und (e) seien beispielsweise Aluminiumchlorid, Zinkchlorid, Zinkacetat, Trifluormethylsulfonsäure, Zinntetrachlorid, 4-Dimethyl-aminopyridin und 4-Pyrrolidinopyridin genannt.

Die Reaktionstemperaturen können bei der Durchführung der erfindungsgemäßen Verfahren (a), (b) und (c) in einem größeren Bereich variiert werden. Im allgemeinen arbeitet man bei Temperaturen zwischen -20 °C und 150 °C, vorzugsweise bei Temperaturen zwischen 0 °C und 100 °C, ganz besonders bevorzugt bei Temperaturen zwischen 20 °C und 80 °C.

Zur Durchführung des erfindungsgemäßen Verfahrens (a), (b) und ( c) setzt man pro Mol an Thieno[3,2-b]pyran-5,7-dion Formel (II) im allgemeinen 0,8 bis 2,5 Mol, vorzugsweise 1,0 bis 1,5 Mol an Säurechlorid der Formel (III) oder 1,0 bis 1,5 Mol an Carbonsäure der Formel (IV) (Verfahren (b)) und gegebenenfalls 0,8 bis 2,5 Mol, vorzugsweise 1,0 bis 1,5 Mol an Reaktionshilfsmittel ein.

Die Reaktionsdurchführung, Aufarbeitung und Isolierung der Reaktionsprodukte erfolgt nach allgemein üblichen Methoden (vgl. auch die Herstellungsbeispiele).

Als Verdünnungsmittel zur Durchführung des erfindungsgemäßen Verfahrens (d) kommen inerte organische Lösungsmittel infrage. Hierzu gehören insbesondere aliphatische, alicyclische oder aromatische Kohlenwasserstoffe, wie beispielsweise Benzol, Toluol, Xylol, Petrolether, Hexan, Cyclohexan, Ether, wie Diethylether, Dioxan, Tetrahydrofuran oder Ethylenglykoldimethyl- oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid oder Sulfone, wie Sulfolan, Alkohole wie Methanol, Ethanol oder Isopropanol, chlorierte Kohlenwasserstoffe wie Chloroform oder Dichlormethan.

Das erfindungsgemäße Verfahren (d) wird vorzugsweise in Gegenwart eines geeigneten Reaktionshilfsmittels durchgeführt. Als solche kommen alle üblicherweise verwendbaren anorganischen und organischen Basen in Frage. Vorzugsweise verwendet man Alkalimetallhydride, -hydroxide, -amide, -alkoholate, -carbonate oder -hydrogencarbonate, wie beispielsweise Natriumhydrid, Natriumamid, Natriumhydroxid, Natriummethylat, Natriumethylat, Kalium-t-butylat, Natriumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU), Oxide von Erdalkalimetallen wie Magnesiumoxid oder Calciumoxid.

Die Base wird in stöchiometrischer Menge oder im Überschuß zugesetzt.

Die Reaktionstemperaturen können bei der Durchführung der erfindungsgemäßen Verfahren ((d)und (e)-in einem größeren Bereich variiert werden. Im allgemeinen arbeitet man bei Temperaturen zwischen -20 °C und 150 °C, vorzugsweise bei Temperaturen zwischen 0 °C und 80 °C,

Zur Durchführung des erfindungsgemäßen Verfahrens (d) setzt man pro Mol an Thieno[3,2-b)pyran-5,7-dion der Formel (I) in welcher $R^1$ für Wasserstoff steht,
im allgemeinen 0,8 bis 2,5 Mol, vorzugsweise 1,0 bis 1,5 Mol an Hydroxylamin oder seiner Ammoniumverbindung $R^3ONH_2$ bzw. $R^3ONH_3{}^{\oplus}W^{\ominus}$ und gegebenenfalls 0,8 bis 2,5 Mol, vorzugsweise 1,0 bis 1,5 Mol an Reaktionshilfsmittel ein. $W^{\ominus}$ steht dahei für ein Anion einer anorganischen Säure wie Chlorid, Bromid, Jodid, Hydrogensulfat oder Phosphat.

Zur Durchführung des erfindungsgemäßen Verfahrens (e) setzt man pro Mol an Thieno[3,2-b]pyran-5,7-dione der Formel (I), in welcher $R^1$ für Wasserstoff steht, im allgemeinen 0,8 bis 2,5 Mol, vorzugsweise 1,0 bis 1,5 Mol an Halogenid der Formel (IX) und gegebenenfalls 0,8 bis 2,5 Mol, vorzugsweise 1,0 bis 1,5 Mol an Reaktionshilfsmittel ein.

Die Reaktionsdurchführung, Aufarbeitung und Isolierung der Reaktionsprodukte erfolgt nach allgemein üblichen Methoden (vgl. auch die Herstellungsbeispiele).

Die Wirkstoffe eignen sich bei günstiger Warmblütertoxizität zur Bekämpfung von pathogenen Endoparasiten die bei Menschen und in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Arten wirksam. Durch die Bekämpfung der pathogenen Endoparasiten sollen Krankheit, Todesfälle und Leistungsminderungen (z.B. bei der Produktion von Fleisch, Milch, Wolle, Häuten, Eiern, Honig usw.) vermindert werden, so daß durch den Einsatz der Wirkstoffe eine wirtschaftlichere und einfachere Tierhaltung möglich ist. Zu den pathogenen Endoparasiten zählen Cestoden, Trematoden, Nematoden, Acantocephalen insbesondere:
Aus der Ordnung der Pseudophyllidea z.B.: Diphyllobothrium spp., Spirometra spp., Schistocephalus spp., Ligula spp., Bothridium spp., Diphlogonoporus spp..

Aus der Ordnung der Cyclophyllidea z.B.: Mesocestoides spp., Anoplocephala spp., Paranoplocephala spp., Moniezia spp., Thysanosomsa spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp..

Aus der Unterklasse der Monogenea z.B.: Gyrodactylus spp., Dactylogyrus spp., Polystoma spp..

Aus der Unterklasse der Digenea z.B.: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp-, Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp. Metorchis spp., Heterophyes spp., Metagonimus spp..

Aus der Ordnung der Enoplida z.B.: Trichuris spp., Capillaria spp., Trichomosoides spp., Trichinella spp..

Aus der Ordnung der Rhabditia z.B.: Micronema spp., Strongyloides spp..

Aus der Ordnung der Strongylida z.B.: Stronylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp., Globocephalus spp., Syngamus spp., Cyathostoma spp., Metastrongylus spp., Dictyocaulus spp., Muellerius spp., protostrongylus spp., Neostrongylus spp., Cystocaulus spp., Pneumostrongylus spp., Spicocaulus spp., Elaphostrongylus spp. Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp..

Aus der Ordnung der Oxyurida z.B.: Oxyuris spp., Enterobius spp., Passalurus spp., Syphacia spp., Aspiculuris spp., Heterakis spp..

Aus der Ordnung der Ascaridia z.B.: Ascaris spp., Toxascaris spp., Toxocara spp., Parascaris spp., Anisakis spp., Ascaridia spp..

Aus der Ordnung der Spirurida z.B.: Gnathostoma spp., Physaloptera spp., Thelazia spp., Gongylonema spp., Habronema spp., Parabronema spp., Draschia spp., Dracunculus spp..

Aus der Ordnung der Filariida z.B.: Stephanofilaria spp., Parafilaria spp., Setaria spp., Loa spp., Dirofilaria spp., Litomosoides spp., Brugia spp., Wuchereria spp., Onchocerca spp..

Aus der Ordnung der Gigantorhynchida z.B.: Filicollis spp., Moniliformis spp., Macracanthorhynchus spp., Prosthenorchis spp..

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Süß- und Salzwasserfische wie z.B. Forellen, Karpfen, Aale, Reptilien, Insekten wie z.B. Honigbiene und Seidenraupe.

Zu Labor- und Versuchstieren gehören Mäuse, Ratten, Meerschweinchen, Goldhamster, Hunde und Katzen.

Zu den Hobbytieren gehören Hunde und Katzen.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal, durch Behandlung der Umgebung oder mit Hilfe wirkstoffhaltiger Formkörper wie z.B. Streifen, Platten, Bänder, Halsbänder, Ohrmarken, Gliedmaßenbänder, Markierungsvorrichtungen.

Die enterale Anwendung der Wirkstoffe geschieht z.B. oral in Form von Pulver, Tabletten, Kapseln, Pasten, Tränken, Granulaten, oral applizierbaren Lösungen, Suspensionen und Emulsionen, Boli, medikiertem Futter oder Trinkwasser. Die dermale Anwendung geschieht z.B. in Form des Tauchens (Dippen), Sprühens (Sprayen) oder Aufgießens (pour-on and spot-on). Die parenterale Anwendung geschieht z.B. in Form der Injektion (intramusculär, subcutan, intravenös, intraperitoneal) oder durch Implantate.

Geeignete Zubereitungen sind:
Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;

Emulsionen und Suspension zur oralen oder dermalen Anwendung sowie zur Injektion; Halbfeste Zubereitungen;
Formulierungen bei denen der Wirkstoff in einer Salbengrundlage oder in einer Öl in Wasser oder Wasser in Öl Emulsionsgrundlage verarbeitet ist;
Feste Zubereitungen wie Pulver, Premixe oder Konzentrate, Granulate, Pellets, Tabletten, Boli, Kapseln; Aerosole und Inhalate, wirkstoffhaltige Formkörper.

Injektionslösungen werden intravenös, intramuskulär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylakohol, Glycerin, Propylenglykol, Polyethylenglykole, N-Methyl-pyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden wie oben bei den Injektionslösungen beschrieben hergestellt, wobei auf steriles Arbeiten verzichtet werden kann.

Lösungen zum Gebrauch auf der Haut werden aufgeträufelt, aufgestrichen, eingerieben, aufgespritzt oder aufgesprüht. Diese Lösungen werden wie oben bei den Injektionslösungen beschrieben hergestellt.

Es kann vorteilhaft sein, bei der Herstellung Verdikkungsmittel zuzufügen. Verdickungsmittel sind: Anorganische Verdickungsmittel wie Bentonite, kolloidale Kieselsäure Aluminiummonostearat, organische Verdickungsmittel wie Cellulosederivate, Polyvinylalkohole und deren Copolymere, Acrylate und Metacrylate.

Gele werden auf die Haut aufgetragen oder aufgestrichen oder in Körperhöhlen eingebracht. Gele werden hergestellt indem Lösungen, die wie bei den Injektionslösungen beschrieben hergestellt worden sind, mit soviel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgieß-Formulierungen werden auf begrenzte Bereiche der Haut aufgegossen oder aufgespritzt, wobei der Wirkstoff die Haut durchdringt und systemisch wirkt.

Aufgieß.Formulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2,2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B.DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Novantisolsäure.

Haftmittel sind z.B. Cellulosederivate, Stärkederivate, Polyacrylate, natürliche Polymere wie Alginate, Gelatine.

Emulsionen können oral, dermal oder als Injektionen angewendet werden.

Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/ Caprinsäure-bigylcerid, Triglyceridgemisch mit Pflanzenfettsäuren der Kettenlänge $C_{8-12}$ oder anderen speziell ausgewählten natürlichen Fettsäuren, Partialglyceridgemische gesättigter oder ungesättigter eventuell auch hydroxylgruppenhaltiger Fettsäuren, Mono- und Diglyceride der $C_8/C_{10}$-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge $C_{16}$-$C_{18}$,Isopropylmyristat, Isopropylpalmitat, Capryl/Caprinsäureester von gesättigten Fettalkoholen der Kettenlänge $C_{12}$-$C_{18}$, Isopropylstearat, Ölsäureoleylester, Ölsäuredecylester, Ethyloleat, Milchsäureethylester, wachsartige Fettsäureester wie künstliches Entenbürzeldrüsenfett, Dibutylphthalat, Adipinsäurediisopropylester, letzterem verwandte Estergemische u.a.

Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt: Wasser, Alkohole wie z.B. Propylenglycol, Glycerin, Sorbitol und ihre Gemische.

Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;

ampholytische Tenside wie Di-Na-N-lauryl-β-iminodipropionat oder Lecithin;

anionaktive Tenside, wie Na-Laurylsulfat, Fettalkoholethersulfate, Mono/Dialkylpolyglykoletherorthophosphorsäureester-monoethanolaminsalz;

kationaktive Tenside wie Cetyltrimethylammoniumchlorid.

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden, Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebene Tenside genannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Tragerstoffe seien genannt alle physiologisch verträglichen festen Inertstoffe. Als solche dienen anorganische und organische Stoffen Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs- und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffen, die gegen pathogene Endoparasiten wirken, vorliegen. Solche Wirkstoffe sind z.B. L-2,3,5,6-Tetrahydro-6-phenylimidazothiazol, Benzimidazolcarbamate, Praziquantel, Pyrantel, Febantel.

Anwendungsfertige Zubereitungen enthalten den Wirkstoff in Konzentrationen von 10 ppm - 20 Gewichtsprozent, bevorzugt von 0,1 - 10 Gewichtsprozent.

Zubereitungen die vor Anwendung verdünnt werden, enthalten den Wirkstoff in Konzentrationen von 0,5 - 90 Gewichtsprozent, bevorzugt von 5 bis 50 Gewichtsprozent.

Im allgemeinen hat es sich als vorteilhaft erwiesen, Mengen von etwa 1 bis etwa 100 mg Wirkstoff je kg Körpergewicht pro Tag zur Erzielung wirksamer Ergebnisse zu verabreichen.

Die erfindungsgemäßen Wirkstoffe können als Defoliants, Desiccants, Krautabtötungsmittel und insbesondere als Unkrautvernichtungsmittel und Wachstumsregulatoren verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich zur selektiven Bekämpfung von monokotylen Unkräutern in monokotylen und dikotylen Kulturen sowohl im Vorauflauf- als auch im Nachauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester,

Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, infrage; ferner auch 2,4-Dichlorphenoxyessigsäure (2,4-D); 4-(2,4-Dichlorphenoxy)-buttersäure (2,4-DB); 2,4-Dichlorphenoxypropionsäure (2,4-DP); 2-[[[[(4,6-Dimethoxypyrimidin-2-yl)-aminol-carbonyl]-amino]-sulfonyl]-methyl]-benzoesäuremethylester (BENSULFURON); 3-Isopropyl-2,1,3-benzothiadiazin-4-on-2,2-dioxid (BENTAZON); 3,5-Dibrom-4-hydroxy-benzonitril (BROMOXYNIL); 5-Amino-4-chlor-2-phenyl-2,3-dihydro-3-oxy-pyridazin (CHLORIDAZON); 2-Chlor-N-{[(4-methoxy-6-methyl-1,3,5-triazin-2-yl)-amino]-carbonyl}-benzolsulfonamid (CHLORSULFURON); N,N-Dimethyl-N'-(3-chlor-4-methylphenyl)-harnstoff (CHLORTOLURON); 5-(2-Chlor-4-(trifluormethyl)phenoxy)-2-nitrobenzoesäure (ACIFLUORFEN); Methyl-5-(2,4-dichlor-phenoxy)-2-nitrobenzoat (BIFENOX); 5-(2-Chlor-4-(trifluormethyl)phenoxy)-N-methylsulfonyl-2-nitrobenzamid (FOMESAFEN); (2-Ethoxy-1-methyl-2-oxo-ethyl)-5-(2-chlor-4-trifluormethyl)-phenoxy)-2-nitrobenzoat (LACTOFEN); 2-[4,5-Dihydro-4-methyl-4-(1-methylethyl-5-oxo-1H-imidazol-2-yl]-5-ethyl-3-pyridincarboxylsäure (IMAZETHAPYR); 2-(4,5-Dihydro-4-methyl-4-(1-methylethyl)-5-oxo-1H-imidazol-2-yl)-3-chinolin-carboxylsäure (IMAZAQUIN); Ethyl-2-((4-chlor-6-methoxy-2-pyrimidinyl)-aminocarbonyl)-aminosulfonyl)-benzoat (CHLORIMURON); 4-Amino-6-t-butyl-3-ethylthio-1,2,4-triazin-5(4H)-on (ETHIOZIN); N-(3-Trifluormethylphenyl)-N,N-dimethylharnstoff (FLUOMETURON); [(4-Amino-3,5-dichlor-6-fluor-2-pyridinyl)-oxy]-essigsäure bzw. deren 1-Methylheptylester (FLUROXYPYR); Methyl-2-[4,5-dihydro-4-methyl-4-(1-methylethyl)-5-oxo-1H-imidazol-2-yl]-4(5)-methylbenzoat (IMAZAMETHABENZ); 3,5-Diiod-4-hydroxybenzonitril (IOXYNIL); N,N-DimethylN'-(4-isopropylphenyl)-harnstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-{[[((4-Methoxy-6-methyl-1,3,5-triazin-2-yl)-amino)-carbonyl]-amino]-sulfonyl}-benzoesäure oder deren Methylester (METSULFURON); N-(1-Ethylpropyl)-3,4-dimethyl-2,6-dinitroanilin (PENDIMETHALIN); 3-(Ethoxycarbonylaminophenyl)-N-(3'-methylphenyl)-carbamat (PHENMEDIPHAM); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON); 2,6-Dinitro-4-trifluormethyl-N,N-dipropylanilin (TRIFLURALIN). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden, Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 10 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

Synthesebeispiele

Beispiel 1

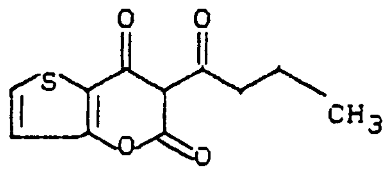

Thieno[3,2-b]-6-butyryl-pyran-5,7-dion
nach Verfahren (b)
18,64 g (0,111 Mol) Thieno[3,2-b]pyran-5,7 dion werden mit 80 ml Buttersäure vorgelegt. Dann werden 30 ml Phosphorylchlorid zugetropft und es wird für 2 Stunden bei 80° C gerührt. Man läßt abkühlen und tropft unter Kühlen vorsichtig 60 ml Ethanol zu. Der ausgefallene Niederschlag wird abgesaugt und mit wenig Ethanol gewaschen.

Zur Reinigung wird aus Ethanol umkristallisiert.
Ausbeute: 10,80 g (40,85 % der Theorie)
Schmelzpunkt: 108-109° C.

Beispiel 2

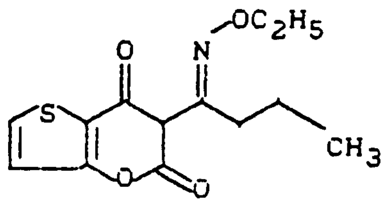

nach Verfahren (d)
Thieno[3,2-b]-6-(l-Ethoxyiminobutyl)-pyran-5,7-dion
4,76 g (0,02 Mol) Thieno[3,2-b]-6-butyril-pyran-5,7-dion werden mit 60 ml Methanol vorgelegt. Dann werden 2,00 g (0,024 Mol) Natriumhydrogencarbonat und 2,34 g (0,24 Mol) Ethoxyaminhydrochlorid zugegeben. Es wird bei Raumtemperatur gerührt und anschließend zur Trockene eingeengt. Der Rückstand wird in 50 ml Methylenchlorid aufgenommen, einmal mit Wasser, zweimal mit gesättigter Natriumhydrogencarbonatlösung, nochmals mit Wasser gewaschen, mit Natriumsulfat getrocknet und das Lösungsmittel im Vakuum entfernt. Es werden 2,8 g (49,8 % der Theorie) Oximether vom Schmelzpunkt Fp: 61-62° C erhalten.

Beispiel 2a

(Methode a)

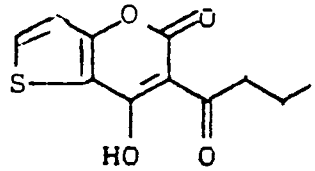

Thieno[3,2-b]-6-butyryl-pyran-5,7-dion

Stufe 1

33,64 g (0,02 Mol) Thieno-[3,2-b]pyran-5,7-dion werden mit 50 ml Methylenchlorid und 26,24 g (0,26 Mol) Triethylamin vorgelegt, dann wird eine Lösung aus 23,60 g (0,22 Mol) Buttersäurechlorid in 40 ml Methylenchlorid zugetropft und für 4 Stunden bei Rückfluß und 12 Stunden bei 20 ° C gerührt. Die Lösung wird in 200 ml Wasser gegossen und die organische Phase mit Magnesiumsulfat getrocknet und das Lösungsmittel in Vakuum entfernt. Das R oh-Produkt wird gesäult (Kieselgel, Methylenchlorid) und das Lösungsmittel in Vakuum entfernt. Es werden 26,5 g (56 % der Theorie) Thieno[3,2-b]-7-butyryloxy-pyran-5-on mit einem Schmelzpunkt Fp.: 55-56 ° C erhalten.

Stufe 2

8,90 g (0,037 Mol) Thieno[3,2-b]-7-butyryloxy-pyran-5-on werden mit 50 ml 1,2-Dichlorethan vorgelegt. Dann werden 9,87 g (0,074 Mol) Aluminiumchlorid zugegeben. Es wird 2 Stunden bei 20 ° C gerührt und dann in Vakuum abrotiert. Der Rückstand wird dann auf 60 g Eis und 60 ml konzentrierte Salzsäure gegossen und abgesaugt. Anschließend wird der Niederschlag in 300 ml 7H NaOH gerührt und nochmals abgesaugt. Dann wird die Mutterlauge angesäuert und der Niederschlag abgesaugt. Es werden 1,20 g (13,5 % der Theorie) Thieno[3,2-b]-6-butyryl-pyran-5,7-dion mit einem Schmelzpunkt Fp.: 108-109 ° C erhalten.

Beispiel 2b (Verfahren c)

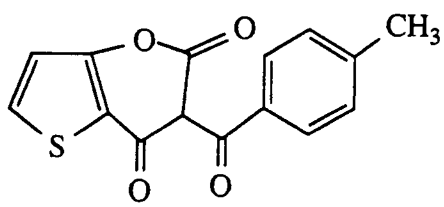

Thieno[3,2-b]-6-(4-methyl)-benzoyl-pyran-5,7-dion

15,46 g (0,10 mol) 4-Methylbenzoylchlorid werden mit 100 ml Toluol und 2,73 g (0,02 mol) Zinkchlorid vorgelegt, dann langsam bei 20 ° C 16,82 g (0,10 mol) Thieno[3,2-b]pyran-5,7-dion zugegeben. Nach 12 Stunden Rühren bei 20 ° C wird das Lösungsmittel im Vakuum entfernt und der Rückstand mit Ether verrührt. Nach Absaugen des Roh-Produktes und aus Acetonitril umkristallisiert. Es werden 13,63 g (48 % der Theorie)

Thieno-[3,2-b]-6-(4-Methyl)-benzoyl-pyran-5,7-dion mit einem Schmelz punkt Fp.: 167-168 ° C erhalten.

Beispiel 2c (Verfahren e)

Thieno[3,2-b]-6-(7-Ethoxyiminopropyl)-7-methoxy-pyran-5-on

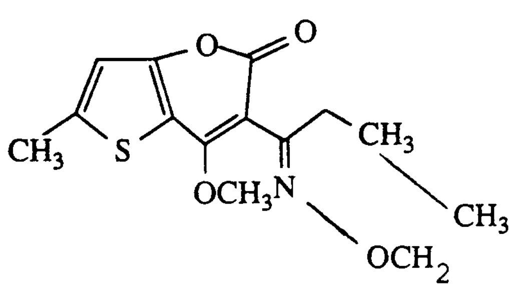

0,50 g (1,78 mmol)

Thieno[3,2-b]-6-(1-Ethoxyiminopropyl)-pyran-5,7-dion werden mit 30 ml Aceton vorgelegt. Dann werden 0.33 g (2,31 mmol) Jodmethan und 0,25 g (1,78 mmol) Kaliumcarbonat zugegeben. Es wird unter Rückfluss für 14 Stunden erhitzt, abgekühlt und anschließend zur Trockne eingeengt. Der Rückstand wird in 100 ml Methylenchlorid aufgenommen, und zweimal mit Wasser gewaschen, mit Magnesiumsulfat getrocknet und

das Lösungsmittel im Vakuum entfernt. Es werden 0,43 g (82 % der Theorie) Produkt erhalten (gelbes Öl). $^1$HNMR (DMSO) δ in 1.05 - 1.35 (6H, 2t, 2 x $CH_3$), 2,50 (3H, s, $CH_3$), 2,70 (sH, q, $CH_2$), 4,10 (3H, s, $OCH_3$), 4,20 (2H, q, $CH_2$), 6,75 (1H, d, Thiophen-H) ppm

Tabelle 1: Herstellungsbeispiele für die Verbindung der Formel (I)

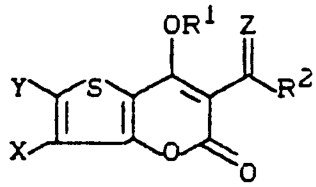

(I)

| Nr. | X | Y | Z | $R^1$ | $R^2$ | Fp[°C] |
|---|---|---|---|---|---|---|
| 3 | H | H | $N-O-CH_3$ | H | Propyl | 87-88 |
| 4 | H | H | N-O 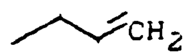 | H | Propyl | |
| 5 | H | H | N-O 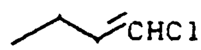 | H | Propyl | |
| 6 | $CH_3$ | H | $N-O-C_2H_5$ | H | Propyl | 104-105 |
| 7 | $CH_3$ | H | N-O 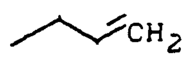 | H | Propyl | |
| 8 | $CH_3$ | H | N-O 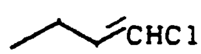 | H | Propyl | |

Tabelle 1: (Fortsetzung)

| Nr. | X | Y | Z | $R^1$ | $R^2$ | Fp[°C] |
|---|---|---|---|---|---|---|
| 9 | H | $CH_3$ | O | H | Propyl | 138 |
| 10 | H | $CH_3$ | $N-OC_2H_5$ | H | Propyl | 86-87 |
| 11 | H | $CH_3$ | $N-OCH_3$ | H | Propyl | 87-88 |
| 12 | H | $CH_3$ | N-O 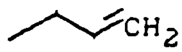 | H | Propyl | |
| 13 | H | $CH_3$ | N-O 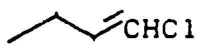 | H | Propyl | |
| 14 | $CH_3$ | $CH_3$ | $N-OC_2H_5$ | H | Propyl | |
| 15 | $CH_3$ | $CH_3$ | N-O 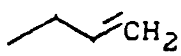 | H | Propyl | |
| 16 | $CH_3$ | $CH_3$ | N-O 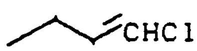 | H | Propyl | |
| 17 | $C_2H_5$ | H | $N-OC_2H_5$ | H | Propyl | |
| 18 | H | $C_2H_5$ | $N-OC_2H_5$ | H | Propyl | |
| 19 | H | Cl | $N-OC_2H_5$ | H | Propyl | |

<u>Tabelle 1</u>: (Fortsetzung)

| Nr. | X | Y | Z | $R^1$ | $R^2$ | Fp[°C] |
|---|---|---|---|---|---|---|
| 20 | Cl | H | $N-OC_2H_5$ | H | Propyl | |
| 21 | Cl | Cl | $N-OC_2H_5$ | H | Propyl | |
| 22 | H | H | O | H | Ethyl | |
| 23 | H | H | N-O 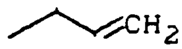 | H | Ethyl | |
| 24 | H | H | N-O 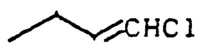 | H | Ethyl | |
| 25 | H | $CH_3$ | O | H | Ethyl | 163-164 |
| 26 | H | $CH_3$ | $N-OC_2H_5$ | H | Ethyl | 131-132 |
| 27 | H | $CH_3$ | N-O 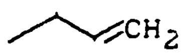 | H | Ethyl | 81-82 |
| 28 | H | $CH_3$ | N-O 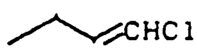 | H | Ethyl | |
| 29 | $CH_3$ | H | N-O 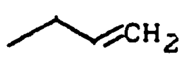 | H | Ethyl | |

Tabelle 1: (Fortsetzung)

| Nr. | X | Y | Z | $R^1$ | $R^2$ | Fp[°C] |
|---|---|---|---|---|---|---|
| 30 | $CH_3$ | H | N-O 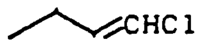 | H | Ethyl | |
| 31 | Cl | Cl | N-O 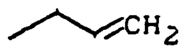 | H | Ethyl | |
| 32 | Cl | H | N-O 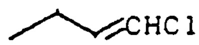 | H | Ethyl | |
| 33 | H | Cl | N-O 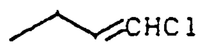 | H | Ethyl | |
| 34 | Br | H | N-O 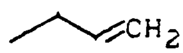 | H | Ethyl | |
| 35 | H | $SCH_3$ | N-O 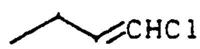 | H | Ethyl | |
| 36 | H | $OCH_3$ | N-O 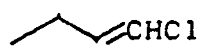 | H | Ethyl | |
| 37 | $CH_3$ | H | O | H | Propyl | 75-76 |
| 38 | $CH_3$ | H | O | H | Ethyl | 89-90 |

Tabelle 1: (Fortsetzung)

| Nr. | X | Y | Z | $R^1$ | $R^2$ |
|---|---|---|---|---|---|
| 39 | H | H | O | H | Methyl |
| 40 | H | H | O | H | Propyl |
| 41 | H | H | O | H | 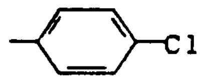 |
| 42 | H | ·H | O | H | 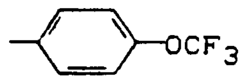 |
| 43 | H | H | O | H | 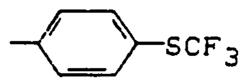 |
| 44 | H | H | O | H | 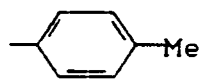 |
| 45 | H | H | O | H | 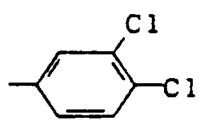 |
| 46 | H | H | O | H | 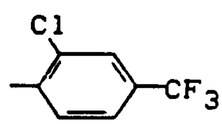 |

Tabelle 1: (Fortsetzung)

| Nr. | X | Y | Z | $R^1$ | $R^2$ |
|---|---|---|---|---|---|
| 47 | H | H | N-$OCH_2$- 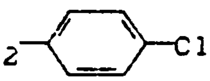 | H | Methyl |
| 48 | H | H | N-$OCH_2$- 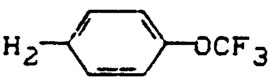 | H | Methyl |
| 49 | H | H | N-$OCH_2$- 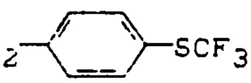 | H | Methyl |
| 50 | H | H | N-$OCH_2$- 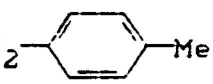 | H | Methyl |
| 51 | H | H | N-$OCH_2$- 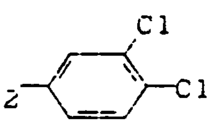 | H | Methyl |
| 52 | H | H | N-$OCH_2$- 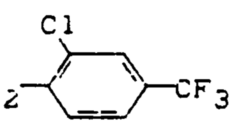 | H | Methyl |
| 53 | H | H | N-$OCH_2$- 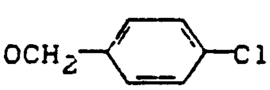 | H | Ethyl |

<u>Tabelle 1</u>: (Fortsetzung)

| Nr. | X | Y | Z | $R^1$ | $R^2$ |
|---|---|---|---|---|---|
| 54 | H | H | 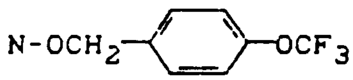 | H | Ethyl |
| 55 | H | H | 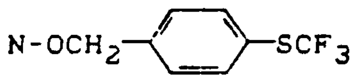 | H | Ethyl |
| 56 | H | H | 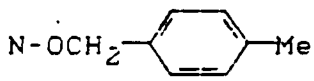 | H | Ethyl |
| 57 | H | H | 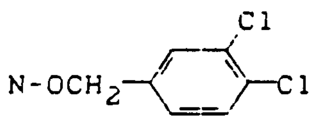 | H | Ethyl |
| 58 | H | H | 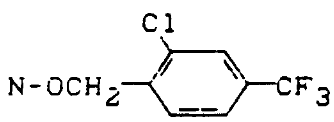 | H | Ethyl |
| 59 | H | H | 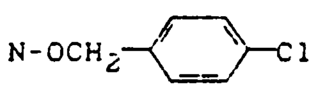 | H | Propyl |
| 60 | H | H | 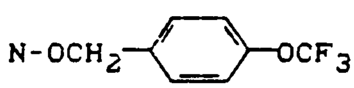 | H | Propyl |

Tabelle 1: (Fortsetzung)

| Nr. | X | Y | Z | $R^1$ | $R^2$ |
|---|---|---|---|---|---|
| 61 | H | H | 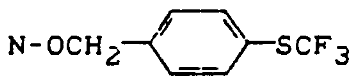 | H | Propyl |
| 62 | H | H | 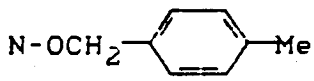 | H | Propyl |
| 63 | H | H | 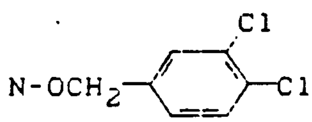 | H | Propyl |
| 64 | H | H | 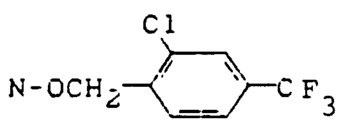 | H | Propyl |
| 65 | H | H | N-O-$CH_3$ | H | 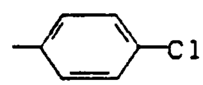 |
| 66 | H | H | N-O-$C_2H_5$ | H | 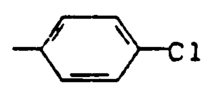 |
| 67 | H | H | N-O-$CH_3$ | H | 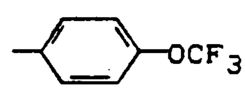 |

Tabelle 1: (Fortsetzung)

| Nr. | X | Y | Z | $R^1$ | $R^2$ |
|---|---|---|---|---|---|
| 68 | H | H | $N-O-C_2H_5$ | H | 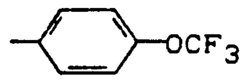 |
| 69 | H | H | $N-O-CH_3$ | H | 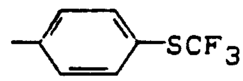 |
| 70 | H | H | $N-O-C_2H_5$ | H | 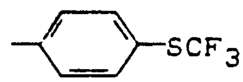 |
| 71 | H | H | $N-O-CH_3$ | H | 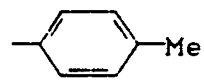 |
| 72 | H | H | $N-O-C_2H_5$ | H | 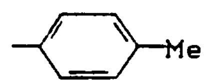 |
| 73 | H | H | $N-O-CH_3$ | H | 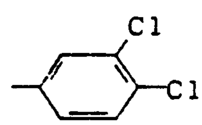 |
| 74 | H | H | $N-O-C_2H_5$ | H | 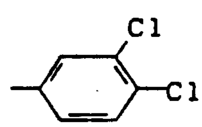 |

Tabelle 1: (Fortsetzung)

| Nr. | X | Y | Z | $R^1$ | $R^2$ |
|---|---|---|---|---|---|
| 75 | H | H | $N-O-CH_3$ | H | 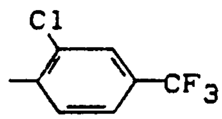 |
| 76 | H | H | $N-O-C_2H_5$ | H | 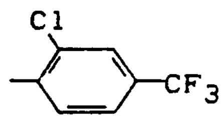 |
| 77 | H | H | O | 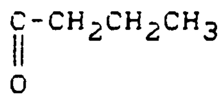 | Propyl |

Beispiele für die Herstellung der Edukte

Synthese von Thieno-[3,2-b]pyran-5,7 dion

Beispiel II-1

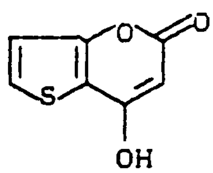

2,07 g (0,00966 Mol) 2-Ethoxycarbonylacetyl-3-hydroxythiophen werden mit 50 ml absolutem Xylol für 1 Stunde zum Rückflußsieden erhitzt. Währenddessen wird langsam das Xylol abdestilliert, wobei das Produkt als Feststoff ausfällt und anschließend abgesaugt wird.

Ausbeute: 0,4 g (22 % der Theorie)
Schmelzpunkt: 223-225 °C

Analog werden dargestellt:

Beispiel II-2

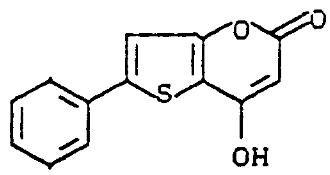

| | |
|---|---|
| $C_{13}H_8O_3S$ Schmelzpunkt: | 214-215 °C |
| NMR ($CDCl_3$): | δ = 5,6 (s, 1H), 7,2 (s, 1H), 7,4 - 7,6 (m, 5H) ppm |
| IR (KBr): | 1640; 1560; 1480; 1400 $cm^{-1}$ |

Beispiel II-3:

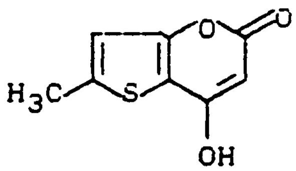

| | |
|---|---|
| $C_8H_6O_3S$ | Schmelzpunkt: 210-212 °C |
| IR (KBr): | 1680; 1560; 1510; 1330 $cm^{-1}$ |
| MS: | 182 (65 %); 140 (100 %); 112 (30 %) |

Beispiel II-4:

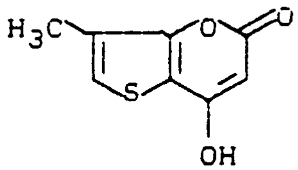

| | |
|---|---|
| $C_8H_6O_3S$ | Schmelzpunkt: 220-221 °C |
| NMR (DMSO): | δ = 2,25 (s, 3H); 5,4 (s, 2H); 7,6 (s, 1H) ppm |

Synthese von 2-Ethoxycarbonylacetyl-3-hydroxy-thiophen

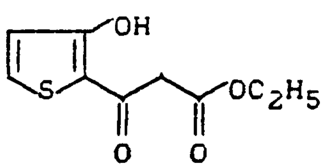

4,8 g (0,0337 Mol) 2-Acetyl-3-hydroxythiophen werden in 150 ml Diethylcarbonat auf 90-100 °C erwärmt. Dann werden in kleinen Stücken 5 g Natrium zugegeben. Es wird 15 Stunden bei 90-100 °C nachgerührt. Zur Zersetzung von Natriumresten wird etwas Ethanol zugegeben. Zur Aufarbeitung wird 2 mal

mit 75 ml Wasser extrahiert, dann wird die wäßrige Phase mit 10 ml konz. HCl angesäuert, 3 mal mit je 50 ml Ether extrahiert, getrocknet und im Vakuum destilliert und die Fraktion zwischen 70 und 100 °C (0,6 mbar, Edukt) sowie zwischen 113 und 118 °C (0,4 mbar Produkt) aufgefangen.

Die Reinigung erfolgt mit Essigester/Cyclohexan (1:1) als Lösungmittel durch Säulenchromatographie.

Ausbeute: 0,5 g (6,9 % der Theorie).

Synthese von 2-Acetyl-3-hydroxythiophen

Beispiel V-1:

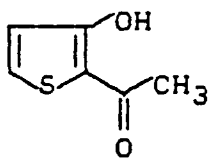

4,65 g (0,086 Mol) Natriummethylat werden in 100 ml Toluol suspendiert. Dann wird eine Suspension von 7,6 g (0,0843 Mol) Acetonylmercaptan in 150 ml Toluol zugegeben. Danach werden 7,1 g (0,0843 Mol) Methylpropiolat zugetropft. Man läßt 1 Stunde unter Rückfluß sieden. Nach dem Abkühlen wird eine Lösung aus 4,5 ml konz. $H_2SO_4$ in 120 ml Wasser zugetropft. Die Toluolphase wird abgetrennt und 3 ml mit je 50 ml Wasser gewaschen, getrocknet und eingeengt. Der Ansatz wird destilliert und die Rohfraktion zwischen 85 °C und 90 °C bei 15,9 mbar aufgefangen. Gewicht des Rohproduktes: 6 g. Das Rohprodukt wird in 48 ml 1 N NaOH eingetragen und mit Ether extrahiert. Die wäßrige Phase wird mit 2 N HCl angesäuert und mit Ether extrahiert. Nach dem Trocknen und Einengen im Vakuum wird mit Ether verrieben.

Ausbeute: 1,2 g (10 % der Theorie)
Schmelzpunkt: 48-50 °C

Analog wurde dargestellt:

Beispiel V-2:

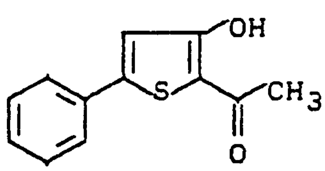

$C_{12}H_{10}O_2S$ Schmelzpunkt: 93-94 °C
NMR ($CDCl_3$): $\delta$ > 2,4 (s,3H), 7,0 (s,1H), 7,4 (m,3H), 7,6 (m,2H), 11,6 (s, 7H) ppm
IR (KBr): 1600; 1480; 1420; 1370; 1340 $cm^{-1}$

Beispiel V-3:

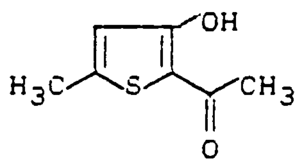

$C_7H_8O_2S$

(Verfahren α)

42,1 g (0,78 Mol) Natriummethylat werden in 1000 ml Toluol suspendiert. Dann werden 70,3 g (0,78 Mol) 2,5-Dihydroxy-2,5-dimethyl-1,4-dithian und 190 g (1,28 Mol) β-Chlor-crotonsäureethylester zugegeben.

Anschließend wird für 2 Stunden zum Rückflußsieden erhitzt. Man läßt abkühlen und gibt eine Lösung von 41,7 ml konz. $H_2SO_4$ in 1100 ml Wasser zu der Reaktionsmischung.

Die organische Phase wird abgetrennt, die wäßrige Phase mit Toluol nachextrahiert. Die vereinigten organischen Phasen werden mit Wasser gewaschen und anschließend über Natriumsulfat getrocknet.

Nach dem Abfiltieren des Trockenmittels wird das Lösungsmittel im Vakuum entfernt. Der Rückstand wird in 450 ml 1 N NaOH-Lösung eingetragen und einmal mit Ether extrahiert. Die wäßrige Phase wird mit 2 N HCl angesäuert und mit Ether extrahiert. Die organischen Phasen werden über Natriumsulfat getrocknet. Nach dem Abtrennen des Trockenmittels und Entfernen des Lösungsmittels im Vakuum wird der Rückstand durch Verreiben mit Ether auskristallisiert.

| | |
|---|---|
| Ausbeute: | 20 g (16 % der Theorie) |
| Schmelzpunkt: | 74 - 75 ° C |
| IR (KBr): | 1490; 1440; 1220; 1040 $cm^{-1}$ |
| NMR (CD $Cl_3$): | δ = 2,3 (s, 3H); 2,45 (d, 3H); 6,5 (d, 1H); 11,7 (s, 1H) ppm |
| MS: | 156 (100 %); 141 (100 %) |

Verfahren β)

Zu einer Lösung von 0,6 g (0,025 Mol) Natriumhydrid in 50 ml absolutem Benzol wird unter Stickstoffatmosphäre eine Lösung von 2,92 g (0,02 Mol) β-Thioacetessigsäureethylester in 10 ml absolutem Benzol getropft.

Anschließend wird für 30 Minuten zum Rückflußsieden erhitzt.

Dann werden 2,78 g (0,03 Mol) Chloraceton, gelöst in 20 ml absolutem Benzol zugetropft und es wird für weitere 3 Stunden zum Rückflußsieden erhitzt. Danach wird der Ansatz auf Eiswasser gegossen und angesäuert. Die organische Phase wird abgetrennt und das Lösungsmittel im Vakuum entfernt.

Der Rückstand wird in 100 ml absolutem DMSO aufgenommen. Unter Stickstoffatmosphäre werden 0,72 g (0,03 Mol) Natriumhydrid zugegeben und anschließend der Ansatz für 2 Stunden gerührt. Danach wird der Ansatz in Wasser eingetragen und angesäuert. Die wäßrige Phase wird mit Ether extrahiert und die organische Phase über Natriumsulfat getrocknet.

Nach dem Entfernen des Lösungsmittels i.Vak. wird das Produkt durch Chromatographie gereinigt (Laufmittel: Essigester/Cyclohexan 1:1).

Ausbeute: 470 mg (15 % der Theorie)

Analog wurden dargestellt:

Beispiel V-4:

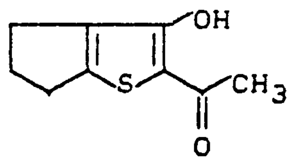

$C_9H_{10}O_2S$(blaßgelbes Öl)

| | |
|---|---|
| NMR ($CDCl_3$): | $\delta$ = 2,3 (s, 3H); 2,4-2,5 (m, 2H); 2,6-2,7 (m, 2H); 2,85-2,95 (m, 2H); 11,8 (s, 1H) ppm |
| MS: | 182 (56 %); 167 (100 %) |

Beispiel V-5:

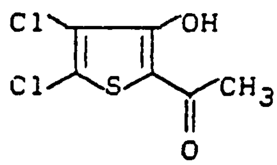

| | |
|---|---|
| $C_6H_4Cl_2O_2S$ Schmelzpunkt: | 84-85 °C |
| NMR ($CDCl_3$): | $\delta$ = 2,15 (s, 3H); 11,9 (s, 1H) ppm |

Beispiel V-6:

Synthese von 2-Acetyl-3-hydroxy-4-methylthiophen

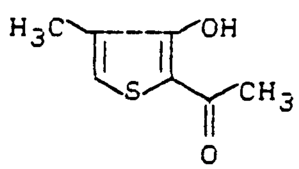

36,0 g (0,227 Mol) 2-Acetyl-3-keto-4-methyl-4,5-dihydrothiophen werden in 260 ml Methylenchlorid vorgelegt. Dann werden bei 0 °C 21,9 ml Sulfurylchlorid in 260 ml Methylenchlorid zugetropft. Während des Zutropfens wird ein Stickstoffstrom in die Lösung eingeleitet, um das entstehende HCl-Gas auszutreiben. Nach beendeter Zugabe wird noch für 30 Minuten bei 10-15 °C gerührt. Dann wird in 600 ml Wasser eingetragen, die organische Phase mit 5 %iger $NaHCO_3$-Lösung extrahiert, getrocknet und im Vakuum eingeengt.

Zur Reinigung wird destilliert.

| | |
|---|---|
| Ausbeute: | 23,1 g (65 % der Theorie) |
| Siedepunkt: | 112 - 115 °C (1,0 mbar) |
| Schmlezpunkt: | 42-43 °C |

Synthese von 2-Acetyl-3-keto-4-methyl-4,5-dihydrothiophen
[(VI), R' = $CH_3$; R = H]

Beispiel VI-1

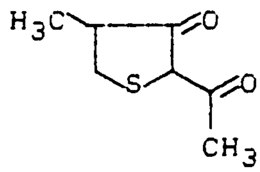

In 1,0 l Toluol werden 26,13 g (- 0,5 Mol) Natriummethylat suspendiert. Dann werden bei 70 °C 88,15 g (0,5 Mol) 3-Acetonylmercaptopropionsäuremethylester zugetropft und es wird für 3 Stunden bei 70 °C nachgerührt. Man läßt abkühlen und trägt in eine Mischung von 750 ml Eiswasser mit 60 ml Eisessig ein. Die organische Phase wird abgetrennt und in 1,5 l 10 %iger Natronlauge eingerührt. Die wäßrige Phase wird mit Eisessig angesäuert und mit Methylenchlorid extrahiert, getrocknet und im Vakuum eingeengt.

Zur Reinigung wird destilliert.

| | |
|---|---|
| Ausbeute: | 38,6 g (53,4 % der Theorie) |
| Siedepunkt: | 63-65 °C (0,5 mbar) |

Synthese von 3-Acetonylmercaptopropionsäuremethylester

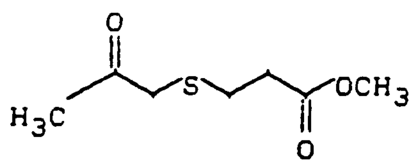

90,1 g (0,5 Mol) 2,5-Dihydroxy-2,5-dimethyl-1,4-dithian werden in 100 ml absolutem Tetrahydrofuran vorgelegt. Dann gibt man 1,0 g Piperidin zu und tropft bei 10-15 °C 94,7 g (0,945 Mol) Acrylsäuremethylester zu, wobei gleichzeitig nochmals zweimal je 0,5 g Piperidin zugesetzt werden. Anschließend wird für 60 Minuten bei 60 °C gerührt. Man läßt abkühlen und trägt in 500 ml Wasser ein.

Es wird mit Methylenchlorid extrahiert, getrocknet und im Vakuum eingeengt. Zur Reinigung wird destilliert. Ausbeute: 147,30 g (81,9 % der Theorie) Siedepunkt: 98-100 °C (2,1 mbar).

Beispiel A

In vivo Nematodentest

Trichostrongylus colubriformis / Schaf

Experimentell mit Trichostrongylus colubriformis infizierte Schafe wurden nach Ablauf der Präpatenzzeit des Parasiten behandelt. Die Wirkstoffe wurden als reiner Wirkstoff in Gelatinekapseln oral appliziert.

Der Wirkungsgrad wird dadurch bestimmt, daß man die mit dem Kot ausgeschiedenen Wurmeier vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzieren (Dosis effectiva).

Geprüfte Wirkstoffe und wirksame Dosierungen (Dosis effectiva) sind aus der nachfolgenden Tabelle ersichtlich.

| Wirkstoff Beispiel Nr. | Dosis effectiva in mg/kg |
|---|---|
| 26 | 100 |

Beispiel B

In vivo Nematodentest

Haemonchus contortus / Schaf

Experimentell mit Haemonchus contortus infizierte Schafe wurden nach Ablauf der Präpatenzzeit des Parasiten behandelt. Die Wirkstoffe wurden als reiner Wirkstoff in Gelatinekapseln oral appliziert.

Der Wirkungsgrad wird dadurch bestimmt, daß man die mit dem Kot ausgeschiedenen Wurmeier vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzeiren (Dosis effectiva).

Geprüfte Wirkstoffe und wirksame Dosierungen (Dosis effectiva) sind aus der nachfolgenden Tabelle ersichtlich:

| Wirkstoff Beispiel Nr. | Dosis effectiva in mg/kg |
|---|---|
| 26 | 50 |

Beispiel C

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

O % = keine Wirkung (wie unbehandelte Kontrolle)
100 % = totale Vernichtung

In diesem Test zeigen die Verbindungen gemäß Herstellungsbeispiele 10, 26, 27 bei guter Verträglichkeit gegenüber Kulturpflanzen, wie z.B. Raps und Soja, starke Wirkung gegen Unkräuter.

Beispiel D

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit gegenüber dem Stand der Technik zeigen in diesem Test z.B. die Verbindungen gemäß den Herstellungsbeispielen 10, 26 und 27 gegen Unkräuter, bei guter Verträglichkeit gegenüber Kulturpflanzen, wie z.B. Weizen, Zuckerrüben und Raps.

**Patentansprüche**

**1.** Substituierte Thieno[3,2-b]pyran-5,7-dione der Formel (I) und ihre tautomeren Formen

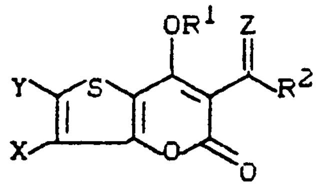

(I)

in welcher

$R^1$ für Wasserstoff, geradkettige oder verzweigte Reste aus der Reihe Alkyl, Alkenyl, Alkinyl Alkylsulfonyl, Alkylcarbonyl, Alkenylcarbonyl, jweils substituiertes oder unsubstituiertes Phenyl, Benzoyl oder Benzoylsulfonyl steht,

$R^2$ für geradkettige oder verzweigte Reste aus der Reihe Alkyl, Alkenyl, Alkinyl, Halogenalkyl, Cycloalkyl, jeweils substituiertes oder unsubstituiertes Phenyl oder Benzyl steht,

X und Y unabhängig voneinander für Wasserstoff, Halogen, Cyano, Nitro, geradkettiges oder verzweigtes Alkyl, Alkenyl, Alkinyl, Halogenalkyl, Cycloalkyl, Alkylcarbonyl, Alkoxycarbonyl, Alkyloxy, Alkylthio, Halogenalkoxy, Halogenalkylthio, Alkylamino, Dialkylamino, jeweils substituiertes oder unsubstituierts Aryl, Aralkyl, Aryloxy oder Arylthio stehen, oder

X und Y gemeinsam mit den angrenzenden C-Atomen einen carbocyclischen Ring bilden, der gegebenenfalls durch Heteroatome unterbrochen ist und

Z für Sauerstoff oder $NOR^3$ steht, wobei

$R^3$ für Wasserstoff und geradkettige oder verzweigte Reste aus der Reihe Alkyl, Alkenyl, Alkinyl, Halogenalkyl, Halogenalkenyl, Halogenalkinyl, Cycloalkyl, substituiertes oder unsubstituiertes Phenyl oder Phenylalkyl steht,

sowie deren verträgliche Salze.

**2.** Substituierte Thieno[3,2-b]pyran-5,7 dione der Formel (I) und ihre tautomeren Formen gemäß Anspruch 1, in welcher

$R^1$ für Wasserstoff, geradkettige oder verzweigte Reste aus der Reihe $C_1$-$C_7$-Alkyl, $C_3$-$C_6$-Alkenyl, $C_3$-$C_6$-Alkinyl, $C_1$-$C_6$-Alkylsulfonyl, $C_1$-$C_6$-Alkylcarbonyl, $C_2$-$C_6$-Alkenylcarbonyl jeweils substituiertes oder unsubstituiertes Phenyl, Benzolsulfonyl oder Benzoyl steht,

$R^2$ für geradkettige oder verzweigte Reste aus der Reihe $C_1$-$C_7$-Alkyl, $C_2$-$C_6$-Alkenyl, $C_2$-$C_6$-Alkinyl, $C_1$-$C_7$-Halogenalkyl mit 1 bis 15 gleichen oder verschiedenen Halogenatomen, $C_3$-$C_7$-Cycloalkyl oder jeweils substituiertes oder unsubstituierts Phenyl oder Benzyl steht,

X und Y unabhängig voneinander für Wasserstoff, Halogen, Cyano, Nitro, geradkettiges oder verzweigtes $C_1$-$C_7$-Alkyl, $C_2$-$C_6$-Alkenyl, $C_2$-$C_6$-Alkinyl, $C_1$-$C_7$-Halogenalkyl mit 1 bis 15 gleichen oder verschiedenen Halognatomen, $C_3$-$C_6$-Cycloalkyl, $C_1$-$C_7$-Alkylcarbonyl, $C_1$-$C_7$-Alkyloxycarbonyl, $C_1$-$C_7$-Alkyloxy, $C_1$-$C_7$-Alkylthio, 1-5 Halogen-$C_{1-4}$-alkyloxy, 1-5 Halogen-$C_{1-4}$-alkylthio, $C_1$-$C_7$-Alkylamino, $C_1$-$C_7$-Dialkylamino, jeweils substituiertes

oder unsubstituiertes Aryl, Aralkyl, Aryloxy oder Arylthio stehen, oder

X und Y gemeinsam mit den angrenzenden C-Atomen einen carbocyclischen Ring bestehend aus 4 bis 8 Kohlenstoffatomen, der gegebenenfalls durch Heteroatome, wie Stickstoff, Sauerstoff oder Schwefel unterbrochen ist, bilden und

Z für Sauerstoff oder $NOR^3$ steht, wobei

$R^3$ für Wasserstoff und für geradkettige und verzweigte Reste aus der Reihe $C_1$-$C_7$-Alkyl, $C_2$-$C_6$-Alkenyl, $C_2$-$C_6$-Alkinyl, $C_1$-$C_7$-Halogenalkyl mit 1 bis 15 gleichen oder verschiedenen Halogenatomen, $C_2$-$C_6$-Halogenalkenyl mit 1 bis 11 gleichen oder ver schiedenen Halogenatomen, $C_2$-$C_6$-Halogenalkinyl mit 1 bis 9 gleichen oder verschiedenen Halogenatomen, $C_3$-$C_6$-Cycloalkyl, oder substituiertes oder unsubstituiertes Phenyl oder Benzyl steht,

wobei als Substituenten an den Aromaten Halogen, Cyano, Nitro, geradkettiges oder verzweigtes Alkyl, Halogenalkyl, Alkoxy, Halogenalkoxy, Alkylthiio, Halogenalkylthio, Alkylsulfinyl, Halogenalkylsulfinyl, Alkylsulfonyl, Halogenalkylsulfonyl, Alkylendioxy, Halogenalkylendioxy in Frage kommen,

sowie deren verträgliche Salze.

**3.** Substituierte Thieno[3,2-b]pyran-5,7-dione der Formel (I) und ihrer tautomeren Formen gemäß Anspruch 1, in welcher

$R^1$ für Wasserstoff, geradkettige oder verzweigte Reste aus der Reihe $C_1$-$C_6$-Alkyl, $C_3$-$C_4$-Alkenyl, $C_3$-$C_4$-Alkinyl, $C_1$-$C_4$-Alkylsulfonyl, $C_1$-$C_4$-Alkylcarbonyl, $C_2$-$C_5$-Alkenylcarbonyl, oder jeweils substituiertes oder unsubstituiertes Phenyl, Benzolsulfonyl oder Benzoyl steht,

$R^2$ für geradkettige oder verzweigte Reste aus der Reihe $C_1$-$C_6$-Alkyl, $C_1$-$C_4$-Alkenyl, $C_2$-$C_4$-Alkinyl, $C_1$-$C_6$-Halogenalkyl mit 1 bis 13 gleichen oder verschiedenen Halogenatomen oder $C_3$-$C_6$-Cycloalkyl,

jeweils substituiertes oder unsubstituiertes Phenyl oder Benzyl steht,

X und Y unabhängig voneinander für Wasserstoff, Halogen, Cyano, Nitro, geradkettiges oder verzweigtes $C_1$-$C_6$-Alkyl, $C_2$-$C_5$-Alkenyl, $C_2$-$C_5$-Alkinyl, $C_1$-$C_6$-Halogenalkyl mit 1 bis 13 gleichen oder verschiedenen Halogenatomen, $C_3$-$C_6$-Cycloalkyl, $C_1$-$C_6$-Alkylcarbonyl, $C_1$-$C_6$-Alkyloxycarbonyl, $C_1$-$C_6$-Alkyloxy, $C_1$-$C_6$-Alkylthio, $C_1$-$C_6$-Alkylamino, $C_1$-$C_6$-Dialkylamino, jeweils substituiertes oder unsubstituiertes Aryl, Aralkyl, Aryloxy oder Arylthio stehen, oder

X und Y gemeinsam mit den angrenzenden C-Atomen einen carbocyclischen Ring bestehend aus 4 bis 8 Kohlenstoffatomen, der gegebenenfalls durch Heteroatome, wie Stickstoff, Sauerstoff oder Schwefel unterbrochen ist, bilden und

Z für Sauerstoff oder $NOR^3$ steht, wobei

$R^3$ für Wasserstoff und für geradkettige oder verzweigte Reste aus de Reihe $C_1$-$C_6$-Alkyl, $C_2$-$C_4$-Alkenyl, $C_2$-$C_4$-Alkinyl, $C_1$-$C_6$-Halogenalkyl mit 1 bis 13 gleichen oder verschiedenen Halogenatomen, $C_2$-$C_4$-Halogenalkenyl mit 1 bis 7 gleichen oder verschiedenen Halogenatomen, $C_2$-$C_4$-Halogenalkinyl mit 1 bis 5 gleichen oder verschiedenen Halogenatomen, $C_3$-$C_6$-Cycloalkyl, oder substituiertes oder unsubstituiertes Benzyl oder für Phenyl steht,

wobei als Substituenten an den Aromaten Halogen, Cyano, Nitro, geradkettiges oder verzweigtes $C_1$-$C_4$-Alkyl, 1-5-Halogen-$C_{1-4}$-alkyl $C_{1-4}$-Alkoxy, 1-5-Halogen-$C_{1-4}$-alkoxy, $C_{1-4}$-Alkylthio, 1-5-Halogen$C_{1-4}$-alkylthio, $C_{1-4}$-Alkylsulfinyl, 1-5-Halogen$C_{1-4}$-alkylsulfinyl, $C_{1-4}$-Alkylsulfonyl, 1-5-Halogen-$C_{1-4}$-alkylsulfonyl, $C_{1-2}$-Alkylendioxy, 1-4-Halogen-$C_{1-2}$-alkylendioxy in Frage kommen,

sowie deren verträgliche Salze mit einwertigen und zweiwertigen Metallkationen, jeweils substituierten oder unsubstituierten Ammoniumionen, Phosphoniumionen, Sulfoniumionen und Sulfoxoniumionen.

**4.** Substituierte Thieno[3,2-b]pyran-5,7-dione der Formel (I) und ihrer tautomeren Formen gemäß Anspruch 1, in welcher

$R^1$ für Wasserstoff, Methyl, Ethyl, n-Propyl, i-Propyl, n-Butyl, i-Butyl, t-Butyl, n-Pentyl, i-Pentyl, Allyl, n-Butenyl, i-Butenyl, Propinyl, Acetyl, Propionyl, Acroyl, Isobutyryl, Pivaloyl, Valeroyl, Phenyl, Benzoyl, 4-Methylbenzoyl, 4-Ethylbenzoyl, 4-n-Propylbenzoyl, 4-Isopropylbenzoyl, 4-n-Butylbenzoyl, 4-i-Butylbenzoyl, 4-t-Butylbenzoyl, 2,4,6-Trimethylbenzoyl, p-Methylphenylsulfonyl, Benzoylsulfonyl steht,

$R^2$ für Methyl, Ethyl, n-Propyl, i-Propyl, n-Butyl, s-Butyl, i-Butyl, t-Butyl, n-Pentyl, i-Pentyl, s-Pentyl, t-Pentyl, neo-Pentyl, n-Hexyl, Cyclopropyl, Cyclobutyl, Cyclopentyl, Cyclohexyl, jeweils substituiertes oder unsubstituiertes Phenyl oder Benzyl steht,

X und Y unabhängig voneinander für Wasserstoff, Fluor, Chlor, Brom, Cyano, Methyl, Ethyl, n-Propyl, Isopropyl, n-Butyl, i-Butyl, s-Butyl, t-Butyl, n-Pentyl, Cyclopropyl, Cyclobutyl, Cyclopentyl, Cyclohexyl, Methoxy, Ethoxy, n-Propyloxy, i-Propyloxy, n-Butyloxy-, i-Butyloxy, t-Butyloxy, n-Pentyloxy oder neo-Pentyloxy, Methylthio-, Ethylthio-, n-Propylthio, i-Propylthio, n-Butylthio-, i-Butylthio, t-Butylthio, n-Pentylthio, Dimethylamino, Diethylamino, Dipropylamino, Dibutylamino, Methoxymethyl, Ethoxymethyl, Methylthiomethyl, Ethylthiomethyl, 1-Methoxyethyl, 2-Methoxythio-ethyl, 1,3-Dimethoxypropyl, 2-Ethoxyethyl, 1-Methyl-2-methylthio-ethyl, 2-Methyl-1-methylthiomethylpropyl, 1-Methyl-butyl, 2-Methyl-1-propenyl, Phenyl, 4-Methylphenyl, 4-Trifluormethylphenyl, 2,4-Dichlorphenyl, 4-Methoxyphenyl, 2,4,6-Trimethylphenyl, 3,5-Dibromphenyl, 4-Methylthiophenyl, 4-Fluorphenyl stehen, oder

X und Y gemeinsam mit den angrenzenden C-Atomen einen carbocyclischen Ring bestehend aus 5 bis 6 Kohlenstoffatomen, der gegebenenfalls durch Heteroatome wie Stickstoff, Sauerstoff oder Schwefel unterbrochen ist, bilden und

Z für Sauerstoff oder $NOR^3$ steht, wobei

$R^3$ für Wasserstoff und für Methyl,Ethyl,n-Propyl,i-Propyl, n-Butyl, i-Butyl, t-Butyl, Allyl, Prop-2-inyl, 3-Fluorpropyl, trans-3-Chlor-prop-2-enyl, trans-But-2-enyl, cis-3-Chlor-prop-2-enyl, But-2-inyl, 2-Methoxyethyl, E-4(4-Fluorphenyl)but-2-enyl, E-4(4-Chlorphenyl)but-2-enyl, E-4(4-tert.-Butylphenyl)but-2-enyl, E-4(4-Trifluorphenyl)but-2-enyl; E-4-Phenyl-but-2-enyl, 2-Thienyl, 3-Thienyl; 5-Chlor-thienyl und gegebenenfalls substituiertes Benzyl steht,

wobei als Substituenten an den Aromaten Halogen, Cyano, Nitro, Methyl, Ethyl, n-Propyl, i-Propyl, n-Butyl, i-Butyl, t-Butyl, Chlormethyl, Fluormethyl, Dichlormethyl, Trichlormethyl, Difluormethyl, Trifluormethyl, Trifluormethoxy, Trifluormethylthio, Methylsulfinyl, Methylsulfonyl, Trifluormethylsulfinyl, Trifluormethylsulfonyl in Frage kommen,

sowie deren verträgliche Salze mit einwertigen Metallkationenen, insbesondere Natrium, Kalium, oder ein Äquivalent eines mehrwertigen Kations, z.B. ein Erdalkalimetallkation, insbesondere Magnesium, Calcium sowie landwirtschaftlich genutzte Kationen wie Mangan-, Kupfer-, Zink- und Eisenkationen sowie Ammonium-, Phosphonium-, Sulfonium- und Sulfoniumkationen wie Ammonium, Tetraalkylammonium, Benzyltrialkylammonium, Trialkylsulfonium oder Trialkylsulfoxonium.

**5.** Verfahren zur Herstellung von Thieno[3,2-b]pyran-5,7-dione dionen der Formel (I) gemäß Anspruch 1

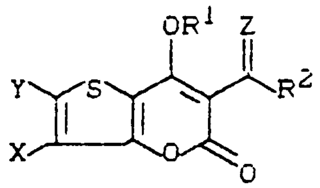

(I)

in welcher

$R^1$ für Wasserstoff, geradkettige oder verzweigte Reste aus der Reihe Alkyl, Alkenyl, Alkinyl Alkylsulfonyl, Alkylcarbonyl, Alkenylcarbonyl, jeweils substituiertes oder unsubstituiertes Phenyl, Benzoyl oder Benzoylsulfonyl steht,

$R^2$ für geradkettige oder verzweigte Reste aus der Reihe Alkyl, Alkenyl, Alkinyl, Halogenalkyl, Cycloalkyl, jeweils substituiertes oder unsubstituiertes Phenyl oder Benzyl steht,

X und Y unabhängig voneinander für Wasserstoff, Halogen, Cyano, Nitro, geradkettiges oder verzweigtes Alkyl, Alkenyl, Alkinyl, Halogenalkyl, Cycloalkyl, Alkylcarbonyl, Alkoxycarbonyl, Alkyloxy, Alkylthio, Halogenalkoxy, Halogenalkylthio, Alkylamino, Dialkylamino, jeweils substituiertes oder unsubstituierts Aryl, Aralkyl, Aryloxy oder Arylthio stehen, oder

X und Y gemeinsam mit den angrenzenden C-Atomen einen carbocyclischen Ring bilden, der

gegebenenfalls durch Heteroatome unterbrochen ist und

Z für Sauerstoff oder $NOR^3$ steht,

wobei

$R^3$ für Wasserstoff und für geradkettige oder verzweigte Reste aus der Reihe Alkyl, Alkenyl, Alkinyl, Halogenalkyl, Halogenalkenyl, Halogenalkinyl, Cycloalkyl, substituiertes oder unsubstituiertes Phenyl oder Phenylalkyl steht,

sowie deren verträgliche Salze,

indem man

a) Thieno[3,2-b]pyran-5,7-dione der Formel (II)

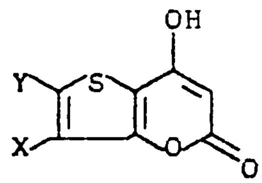

(II)

in welcher

X und Y die oben angegebenen Bedeutungen haben mit einem Säurechlorid der Formel (III)

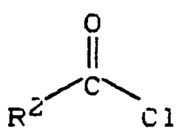

(III)

in welcher

$R^2$ die oben angegebene Bedeutung hat,

gegebenenfalls in Gegenwart einer Base und gegebenenfalls in Gegenwart eines Verdünnungsmittels zum Enolester umsetzt und anschließend gegebenenfalls in Gegenwart eines Reaktionshilfsmittels in gleicher oder einem anderen Verdünnungsmittel bei einer Temperatur zwischen 0°C und 100°C umlagert, oder

b) indem man die entsprechenden Thienopyran-2,4-dione gemäß der allgemeinen Formel (II a) einer Carbonsäure der allgemeinen Formel (IV)

$R^2$-COOH (IV)

in welcher

$R^2$ die oben angegebene Bedeutung hat,

gegebenenfalls in Gegenwart eines Verdünnungsmittels und gegebenenfalls in Gegenwart eines wasserabspaltenden Reagenz und gegebenenfalls in Gegenwart eines Reaktionshilfsmittels bei einer Temperatur zwischen 0°C und 100°C umsetzt,

oder wenn man

c) Thieno[3,2-b]pyran-5,7-dione der Formel (II) mit Säurechloriden der Formel (III) in Gegenwart einer Lewis-Säure in einem Reaktionsschritt zu Derivaten der allgemeinen Formel (I),

in welcher Z für Sauerstoff steht und $R^1$ für Wasserstoff steht,

umsetzt,

oder

d) indem man für den Fall, daß in Formel (I) Z für $=NOR^3$ steht Thieno[3,2-b]pyran-5,7-dione der Formel (I),

worin Z für Sauerstoff steht und die übrigen Reste die oben angegebene Bedeutung haben,

mit einem entsprechenden Hydroxylamin oder seinen Ammoniumverbindungen $R^3ONH_2$ bzw.

$R^3ONH_3{}^{\oplus}W^{\ominus}$

wobei

$W^{\ominus}$ für ein Anion-Äquivalent einer anorganischen Säure und $R^3$ die oben angegebene Bedeutung hat

bei Temperaturen zwischen 0 und 80 ° C umsetzt,

oder

e) indem man für den Fall, daß in Formel (I) $R^1$ für geradkettige oder verzweigte Reste aus der Reihe Alkyl, Alkenyl, Alkinyl, Alkylsulfonyl, Alkylcarbonyl, Alkenylcarbonyl, jeweils substituiertes oder unsubstituiertes Phenyl, Benzoyl oder Benzoylsulfonyl steht, und $R^2$, X, Y und Z die obengenannte Bedeutung haben, entsprechende Verbindungen der Formel (I),

in welcher $R^1$ für Wasserstoff steht mit Halogeniden der Formel (IX), in welcher V und $R^1$ die obengenannte Bedeutung haben,

umsetzt.

**6.** Endoparasitizide sowie herbizide Mittel und Pflanzenwachstumsregulatoren, gekennzeichnet durch einen Gehalt an mindestens einem substituierten Thieno-[3,2-b]pyran-5,7-dion der Formel (I) gemäß Anspruch 1.

**7.** Verfahren zur Bekämpfung von Unkräutern, dadurch gekennzeichnet, daß man substituierte Thieno[3,2-b]pyran-5,7-dione der Formel (I) gemäß Anspruch 1 auf Unkräuter und/oder ihren Lebensraum einwirken läßt.

**8.** Verwendung von substituierten Thieno[3,2-b]pyran-5,7-dionen der Formel (I) gemäß Anspruch 1 zur Bekämpfung von Endoparasiten sowie von Unkräutern.

**9.** Verfahren zur Herstellung von endoparasitiziden sowie herbiziden Mitteln, dadurch gekennzeichnet, daß man substituierte Thieno[3,2-b]pyran-5,7-dione Formel (I) gemäß Anspruch 1 mit Streckmitteln und/oder oberflächenaktiven Substanzen vermischt.

**10.** Verwendung von Thieno[3,2-b]pyran-5,7-dione der Formel (I) gemäß Anspruch 1 zur Herstellung von endoparasitiziden Mitteln.

**11.** 2-Acetyl-3-keto-4,5-dihydrothiophene der Formel (VI)

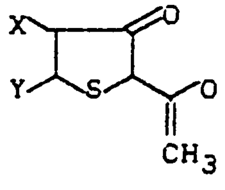

(VI)

in welcher

X und Y unabhängig voneinander für Wasserstoff, Halogen, Cyano, Nitro, geradkettiges oder verzweigtes Alkyl, Alkenyl, Alkinyl, Halogenalkyl, Cycloalkyl, Alkylcarbonyl, Alkoxycarbonyl, Alkyloxy, Alkylthio, Alkylamino, Dialkylamino, gegebenenfalls substituiertess oder unsubstituiertes Aryl, Aralkyl, Aryloxy oder Arylthio stehen, oder

X und Y gemeinsam einen carbocyclischen Ring bilden, der gegebenenfalls durch Heteroatome unterbrochen ist,

wobei als Substituenten an den Aromaten, Halogene, Cyano, Nitro, geradkettiges oder verzweigtes Alkyl, Halogenalkyl, Alkoxy, Halogenalkoxy, Alkylthio, Halogenalkylthio, Alkylsulfinyl, Halogenalkylsulfinyl, Alkylsulfonyl, Halogenalkylsulfonyl, Alkylendioxy, Halogenalkylendioxy in Frage kommen.

Europäisches Patentamt

# EUROPÄISCHER RECHERCHENBERICHT

Nummer der Anmeldung

EP 91 11 7518

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | DE-A-1 964 803 (FIRMENICH)<br>* Beispiel 3 * | 11 | C07D495/04<br>C07D333/32<br>A61K31/38<br>A01N43/90<br>//(C07D495/04, 333:00,311:00) |
| X | FR-A-896 345 (I.G. FARBENINDUSTRIE AG)<br>* Anspruch 1; Beispiel 1 * | 11 | |
| D,A | EP-A-0 345 635 (BASF AG)<br>* Seite 10, Zeile 44 - Seite 12, Zeile 55 *<br>* Seite 16; Anspruch 1 * | 1,6 | |
| A | EP-A-0 241 834 (BAYER AG)<br>* Seite 7, Zeile 27 - Zeile 37; Anspruch 1 * | 1,6 | |
| A | DE-A-3 701 298 (BASF AG)<br>*Seite8,Verbindungen 55 ,56;Anspruch 1;Seite2,Zeile66-Zeile68* | 1,6 | |
| A | WO-A-8 804 652 (NIPPON SODA CO. LTD.)<br>*Zusammenfassung* | 1,6 | |
| P,A | EP-A-0 411 419 (BAYER AG)<br>* Seite 11, Zeile 38 - Seite 12, Zeile 32; Anspruch 1 * | 1,6 | **RECHERCHIERTE SACHGEBIETE (Int. Cl.5)**<br>C07D<br>A01N |

Der vorliegende Recherchenbericht wurde für alle Patentansprüche erstellt

| Recherchenort | Abschlußdatum der Recherche | Prüfer |
|---|---|---|
| DEN HAAG | 23 JANUAR 1992 | HEYWOOD C.J. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)